# EUROPEAN PATENT APPLICATION

(11) **EP 4 745 131 A1**
(43) Date of publication of application: **20.05.2026**
(21) Application number: 24838699.7
(22) Date of filing: 05.07.2024
(51) Int. Cl.: C07D 401/14, C07D 403/04, C07D 403/14, C07D 417/14, A61K 31/506, A61P 25/00, A61P 25/16, A61P 25/28, A61P 3/00, A61P 3/10, A61P 3/06, A61P 3/04, A61P 35/00, A61P 35/02, A61P 13/12, A61P 11/00, A61P 1/16, A61P 9/00, A61P 17/00

(54) **PYRIMIDINAMINE NUAK INHIBITOR AS WELL AS PREPARATION METHOD THEREFOR AND USE THEREOF**

(30) Priority: 13.07.2023 CN 202310860150
(71) Applicant: Technoderma Medicines Inc., Chengdu, Sichuan 610219 (CN)
(72) Inventor: FANG, Wenkui Ken, California 92618 (US); LI, Guanqun, Oregon 97239 (US); CAI, Yuting, Chengdu, Sichuan 610219 (CN); WANG, Zengquan, Nevada 89113 (US)
(74) Representative: Dantz, Jan Henning
(86) International application number: PCT/CN2024/103752
(87) International publication number: WO 2025/011446

(57) **Abstract**

Provided in the present application are pyrimidinamine compounds, characterized in that the pyrimidinamine compounds are compounds represented by formula I, or stereoisomers, tautomers, isotope derivatives, hydrates, solvates, prodrugs and pharmaceutically acceptable salts thereof. The pyrimidinamine compounds of the present application have excellent NUAK1/NUAK2 kinase inhibitory activity, and thus can be used for preventing or treating the following diseases by means of inhibiting NUAK1/NUAK2: neuropsychiatric diseases, metabolic diseases, tumors, visceral fibrosis diseases and skin fibrosis diseases.

## Description

### CROSS-REFERENCE TO RELATED APPLICATIONS

The present application claims priority to Chinese Patent Application No. 202310860150.9, filed on July 13, 2023, which is incorporated herein by reference in its entirety.

### TECHNICAL FIELD

The present disclosure relates to the field of small molecule compounds, specifically to a pyrimidinamine-based NUAK inhibitor and a preparation method and use thereof. The compound, by inhibiting NUAK1/NUAK2, is useful for the prevention or treatment of the following diseases: neuropsychiatric diseases, metabolic diseases, tumors, visceral fibrotic diseases, and skin fibrotic diseases, or used solely for alleviating traumatic and postoperative scars.

### BACKGROUND

Protein kinases are a group of important functional proteins participating in the regulation of metabolism, polarity, growth, division, and differentiation of cells, and the human genome encodes more than 500 types of protein kinases that can transfer phosphate groups from ATP (adenosine triphosphate) to specific serine, threonine, or tyrosine residues of substrate proteins for phosphorylation. Most protein kinases are serine/threonine kinases, and there are less than 100 types of tyrosine kinases. Adenosine monophosphate-activated protein kinase (AMPK) belongs to serine/threonine kinases (STK) and is an important regulator of cellular energy homeostasis in mammals. AMPK can regulate glucose and lipid metabolism, cell proliferation, and cell polarity by sensing a change in an intracellular AMP (Adenosine monophosphate)/ATP or ADP (adenosine diphosphate)/ATP ratio under metabolic stress (for example, hypoxia or heat shock), and therefore, can be referred to as a metabolic sensor protein. AMPK is evolutionarily highly conserved and a heterotrimeric protein consisting of an α subunit (containing a kinase domain, KD), a β subunit (regulating kinase activity), and a γ subunit. The γ subunit contains four cystathionine-β-synthase (CBS) domains and is responsible for detecting changes in the intracellular AMP/ATP and ADP/ATP ratios (Hardie DG, Trends in Cell Biology. 2016, 26:190).

AMPK dysfunction can cause a variety of diseases such as obesity, diabetes, inflammatory diseases and tumors, and therefore, regulation of AMPK function by the body is critical. First of all, phosphorylation of threonine 172 (T172) on the α subunit by upstream kinases is required for AMPK activation. Three types of upstream kinases of AMPK have been found by now, namely, liver kinase B1 (LKB1), Ca²⁺/calmodulin-dependent PK kinase 2 (CaMKK2) and transforming growth factor-β-activated kinase 1 (TAK1). Corresponding to the activation of AMPK via phosphorylation of the T172 site by the upstream kinase, three protein phosphatases are found to inhibit the activity of AMPK by removing a phosphate group from T172, including protein phosphatase 2A (PP2A), protein phosphatase 2C (PP2C) and Mg²⁺/Mn²⁺-dependent protein phosphatase 1E (PPM1E). When cells are in a low-energy state (high AMP/ATP ratio or ADP/ATP ratio), the α subunit KD and γ subunit of AMPK are tightly cross-linked, while the β subunit is myristoylated, preventing protein phosphatases from accessing the T172 site and thus maintaining AMPK activation. In addition, when cells are in a high-energy state, KD and γ subunit are detached, and T172 is exposed to phosphatase, thereby causing inactivation of AMPK (Steinberg GR, Nature Reviews Drug Discovery. 2019, 18:527).

In addition to AMPK upstream kinases and protein phosphatases that can activate and inactivate AMPK respectively, there are also a type of AMPK-related kinases (ARK) participating in the regulation of AMPK functions, and a total of 12 ARKs (BRSK1, BRSK2, NUAK1, NUAK2, QIK, QSK, SIK, MARK1, MARK2, MARK3, MARK4 and MELK) have been found by now and all belong to serine/threonine kinases. Kinase domains of ARKs have high homology with the α subunit of AMPK. All ARKs can be activated by LKB1 except MELK, their kinase-activating phosphorylation sites are equivalent to AMPK T172, and ARKs also functionally participate in the regulation of metabolism, proliferation and polarity of cells. However, unlike AMPK with regulatory subunits, ARKs cannot be directly regulated by the intracellular AMP/ATP ratio (Bright NJ, Acta Physiologica. 2009, 196:15).

ARKs are classified into several ARK subfamilies by different protein structures and functional characteristics. NUAK subfamily (Nu (novel) and AMPK-related kinase) of ARKs includes two members of NUAK1 (initially named ARK5) and NUAK2 (initially named sucrose nonfermenting-like/AMPK related kinase, SNARK). Amino acid sequences of the two members are approximately 55% homologous, and molecular weights of NUAK1 and NUAK2 estimated according to amino acid sequences are 76 kDa and 69 kDa respectively. They share highly similar protein structures, with an N-terminal kinase domain and a C-terminal ubiquitin-binding domain, and currently, it is yet unclear whether NUAK is a heterotrimeric structure like AMPKs. NUAK1 and NUAK2 are expressed in most tissues, an expression level of NUAK1 in organs and tissues such as brain, skin, muscle, upper gastrointestinal tract and endocrine is significantly higher than that in other parts, organs and tissues with the highest expression level of NUAK2 are the gastrointestinal tract, female reproductive system, skin, bone, brain, endocrine and the like, and the expression of NUAK2 shows more tissue specificity. It should be noted that NUAK1 and other ARKs and AMPK proteins are mainly distributed in cytoplasm, while NUAK2 is mainly distributed in nucleus, and there are indications that NUAK2 participates in the expression of genes related to metabolic stress as a transcriptional regulator (Sun X, J of Molecular Endocrinology. 2013, 51:R15).

As serine/threonine kinases, NUAK1 and NUAK2 can phosphorylate a variety of protein substrates, including proteins participating in cell signaling and metabolism, cell proliferation, cell apoptosis and autophagy, and cytoskeletal tissues. As it is known, NUAK1 can phosphorylate AMPK, LATS1/2, p53 tumor suppressor protein, and myosin phosphatase target subunit 1 (Mypt1) regulating actin cytoskeletal tissue. NUAK2 can phosphorylate transcription factors Gli3 and FoxO1 of Hedgehog signaling pathway, kinesin light chain 1 (KLC1), Rho GDP dissociation inhibitor α (Rho GDIα), and the like.

The functional regulation of NUAK1 and NUAK2 relates to a variety of intracellular signaling systems. In addition to being activated via phosphorylation by LKB1 (T211, equivalent to T172 of AMPK) and activated by Ca²⁺/PKC, NUAK1 is the only member of the AMPK-related protein family that can be activated by Akt, and an increase in the activity of NUAK1 is also found in the activation of signaling pathways of some growth factors such as insulin-like growth factor 1 (IGF1). Contraction states of skeletal muscle cells are also accompanied by increased activity of NUAK1. Both NUAK1 and NUAK2 can be activated by LKB1 (T211/NUAK1, T208/NUAK2, equivalent to T172 of AMPK), but NUAK2 can be autophosphorylated, and NUAK2 can interact with ubiquitin-specific protease 9, X-linked (USP9X), which is a deubiquitinase and can maintain activity of NUAK2. Different intracellular stimuli such as low osmotic pressure, DNA damage, oxidation and nutrient deficiencies can all lead to the activation of NUAK2, and the activation of NUAK2 is also found in the contraction of skeletal muscle cells (Brooks D, Data in Brief. 2022, 43:108482).

A number of studies have revealed that NUAK plays an important role in pathogenesis of diseases such as metabolic diseases, tumors, neurodegenerative diseases and fibrotic diseases, homozygous NUAK1/NUAK2 knockout mice basically die in the embryonic period, mice with heterozygous NUAK1 gene deleted exhibit systemic and nervous tissue hypoplasia (for example, cerebral cortex and peripheral neurons), heterozygous gene mutation of human NUAK1 is associated with autism spectrum disorders (ASD), cognitive deficits, attention deficit/hyperactivity disorder (AD/HD) and schizophrenia. Deletion of the human NUAK2 gene causes anencephaly, which is a severe neural tube malformation that causes defective fetal development and whose pathogenesis is related to loss of YAP function (Bonnard C, J Exp Med. 2020, 217:e20191561). Skeletal muscle cell-specific knockout of the NUAK1 gene can avoid high-fat diet-induced subclinical diabetes. However, a phenotype of mice with heterozygous NUAK2 gene deleted is similar to a series of clinical manifestations of human type II diabetes mellitus with obesity, and these phenomena may be related to imbalance of cellular autophagy mechanism (Bennison SA, Cellular Signaling. 2022, 100:110472; Blazejewski SM, Scientific Reports. 2011, 11:8156).

Activation of NUAK1 caused by Akt and other protein kinases can promote survival of tumor cells in an environment with energy deficiency and protect tumor cells from apoptosis, and NUAK2 also inhibits, through a similar mechanism, apoptosis induced by TNF α and CD95. In addition, after activation, NUAK1 promotes invasion and metastasis of tumor cells by upregulating the matrix metalloproteinases (MMP), while NUAK2 participates in occurrence of tumor metastasis by enhancing tumor cell motility (Hou X, Oncogen. 201, 30:2933; Chen Y, Cell Death and Disease. 2020, 11:712; Molina E, Cells. 10:2760; Humbert N, The EMBO Journal. 2010, 29:376). A lot of evidence reveals that NUAK1 and NUAK2 play a role in tumor formation and metastasis, NUAK2 has a stronger function in promoting tumor formation and metastasis than NUAK1, and NUAK2 inhibitors should be more likely to become new targeted antitumor drugs than NUAK1 inhibitors.

Recent studies have revealed that NUAK plays a critical role in development of tissue fibrosis through interaction with transforming growth factor-β (TGF-β) signaling pathway. First, TGF-β can upregulate gene transcription of NUAK1 and NUAK2 in a variety of epithelial cells such as keratinocytes and human dermal fibroblasts, while inactivation of MAPK (ERK1/2 and p38) signals inhibits expression of TGF-β-dependent NUAK2. In addition, NUAK2 inhibits degradation of SMAD3 in cells by binding to the structurally crosslinking domain and MH2 domain of the intracellular signaling mediator SMAD3 protein of TGF-β. A similar mechanism exists between NUAK2 and TβRI. TGF-β-induced expression of genes encoding pro fibrotic molecules such as fibronectin (FN), plasminogen activator inhibitor 1 (PAI1) and tissue inhibitor of metalloproteinase-1 (TIMP1) depends on the presence of NUAK2. These findings indicate that NUAK2 promotes fibrogenesis. Studies have also revealed that NUAK1 promotes fibrosis of kidney, lung and liver by upregulating two signaling pathways of TGF-β and YAP, and in animal experiments, inhibition of NUAK1 can alleviate scars and mature scar tissues caused by new trauma (Gill MK, Nature Communications. 2018. 9:3510). Interestingly, studies have revealed that when expression of genes encoding FN in keratinocytes with NUAK1 gene knocked out is upregulated, NUAK1 may inhibit fibrosis by influencing TGF-β signaling through a negative feedback mechanism (van de Vis RAJ, Cancers. 2021, 13:3377). In general, inhibition of NUAK may effectively inhibit fibrosis processes of tissues and organs involved.

Given their structural and functional properties, kinases have long been ideal targets for small-molecule drugs, and NUAK1 and NUAK2 are involved in the occurrence of a variety of diseases, some NUAK inhibitors have been in an early stage of development by now, but their efficacy and safety are yet undetermined (Banerjee S, The Biochemical Journal. 2014, 457:215). Therefore, development of selective NUAK inhibitors and dual-target NUAK1/2 inhibitors is expected to provide a new direction for innovative treatments of neuropsychiatric diseases (for example, Parkinson's disease and Alzheimer's disease), metabolic diseases (for example, diabetes, hyperlipidemia and obesity), tumors (for example, liver cancer, leukemia, lymphoma and tumor metastasis), visceral fibrotic diseases (for example, liver cirrhosis, renal fibrosis, pulmonary fibrosis and post-myocarditis sequelae), and skin fibrotic diseases (for example, scleroderma, keloids and hypertrophic scars), or solely for alleviating traumatic and postoperative scars. Developing next-generation NUAK inhibitors has significant potential clinical application value.

### SUMMARY OF THE INVENTION

An objective of the present disclosure is to obtain an effective NUAK1/NUAK2 inhibitor that is useful for preparing medicaments for prevention or treatment of the following diseases: neuropsychiatric diseases (for example, Parkinson's disease and Alzheimer's disease), metabolic diseases (for example, diabetes, hyperlipidemia and obesity), tumors (for example, liver cancer, leukemia, lymphoma and tumor metastasis), visceral fibrotic diseases (for example, liver cirrhosis, renal fibrosis, pulmonary fibrosis and post-myocarditis sequelae), and skin fibrotic diseases (for example, scleroderma, keloids and hypertrophic scars), or solely for alleviating traumatic and postoperative scars.

To achieve the foregoing objective, an aspect of the present disclosure provides a pyrimidinamine-based compound, where the pyrimidinamine-based compound is a compound represented by Formula I shown below, or a stereoisomer, a geometric isomer, a tautomer, an isotopic derivative, a hydrate, a solvate, a prodrug, or a pharmaceutically acceptable salt thereof; 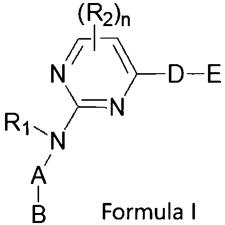
wherein A is a phenyl group optionally substituted by m R₃ groups, or a 5-membered heteroaryl group optionally substituted by p R₄ groups;
B is an optionally substituted 3- to 10-membered cycloalkyl group or an optionally substituted 3- to 10-membered heterocycloalkyl group;
the substituent on A or B is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;
D is 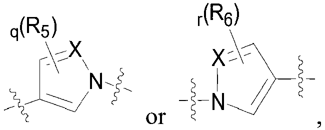 X is N or CH, and when X is CH, the H atom may be substituted by R₅ or R₆;
E is -C(O)-NHR₇R₈, -C(O)-R₇R₈, -NR₇-C(O)R₈, -NR₇-S(O)₂-R₈, an optionally substituted C3-C8 heterocycloalkyl-S(O)₂-R₈, or an optionally substituted C3-C8 cycloalkylS(O)₂-R₈; R₈ is an optionally substituted C1-C8 alkyl group, an optionally substituted C3-C8 cycloalkyl group, or an optionally substituted C3-C8 cycloalkyl C1-C8 alkyl group, and the substituent on the cycloalkyl group or heterocycloalkyl group is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group.
R₁ and R₇ are each independently H or an optionally substituted C1-C8 alkyl group;
R₂, R₃, R₄, R₅, and R₆ are each independently halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, or an optionally substituted C1-C8 alkylamino group;
the substituent on the C1-C8 alkyl group is one or more selected from the group consisting of halogen, an amino group, a hydroxyl group, a nitro group, a cyano group, and a sulfhydryl group; and
n is 0, 1, or 2; m is 0, 1, 2, 3, or 4; and p, q, and r are each 0, 1, 2, or 3.

In one set of embodiments, E is one of the following structures: 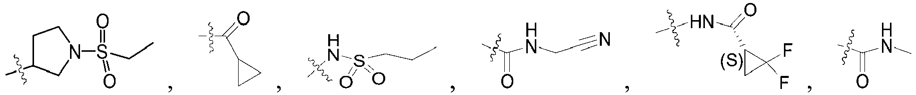 or

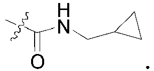

In one set of embodiments, B is an optionally substituted 3- to 6-membered heterocycloalkyl group, preferably, B is an optionally substituted 5- to 6-membered heterocycloalkyl group, and more preferably, B is an optionally substituted piperidinyl group, an optionally substituted piperazinyl group, or an optionally substituted pyrrolidinyl group.

Further, B is one of the following formulae: 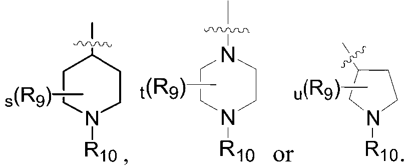
R₉ is halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, or an optionally substituted C1-C8 alkylamino group;
R₁₀ is H or an optionally substituted C1-C8 alkyl group;
the substituent on the C1-C8 alkyl group is one or more selected from the group consisting of halogen, an amino group, a hydroxyl group, a nitro group, a cyano group, and a sulfhydryl group; and
s and t are each 0, 1, 2, 3, or 4; u is 0, 1, 2, or 3.

Further, B is one of the following formulae:

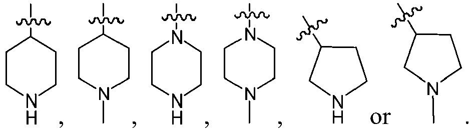

In one set of embodiments, A is one of the following optionally substituted moieties: 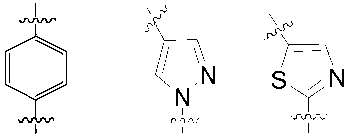 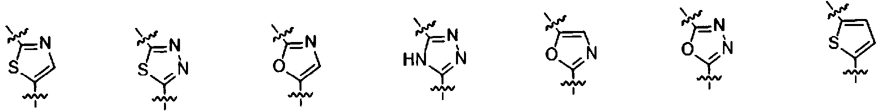 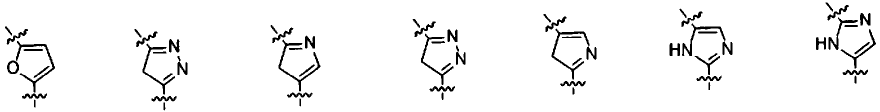 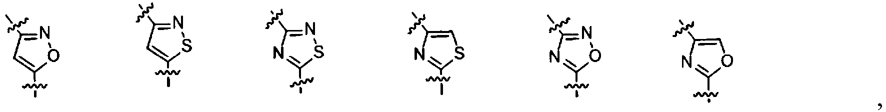 where the upper position of A is attached to the amino group, and the lower position of A is attached to the moiety B.

Further, A is an optionally substituted phenyl group; still further; A is substituted with a methoxy group, preferably at the ortho-position to the amino group.

In one set of embodiments, R₂ is chloro, preferably at the para-position to the amino group.

In one set of embodiments, the compound represented by Formula I is one of the following compounds:

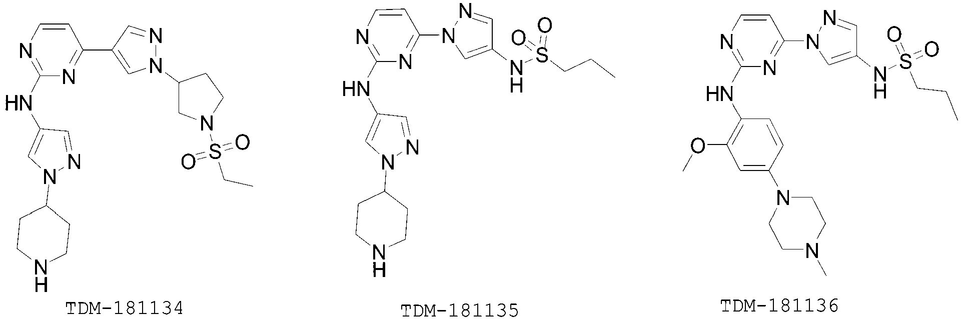

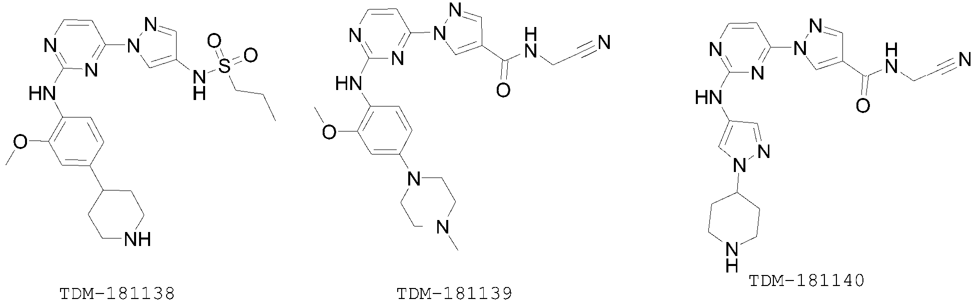

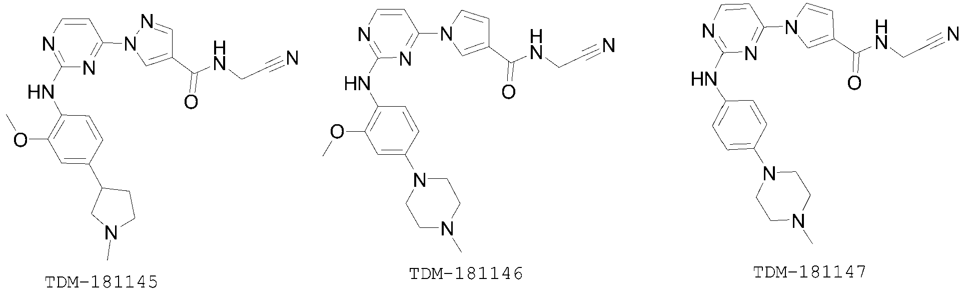

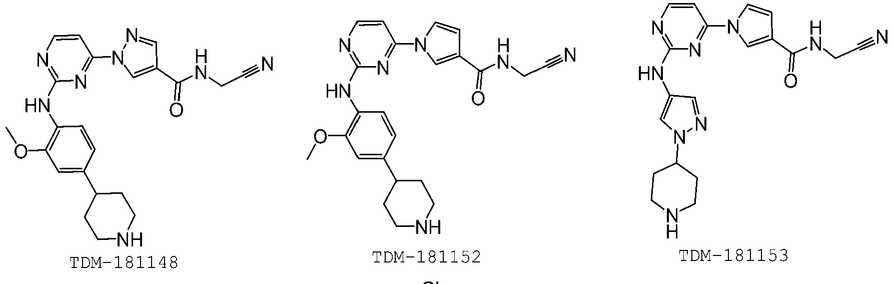

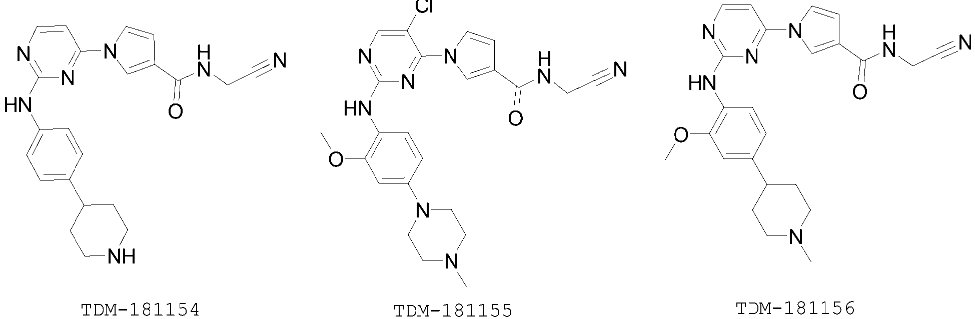

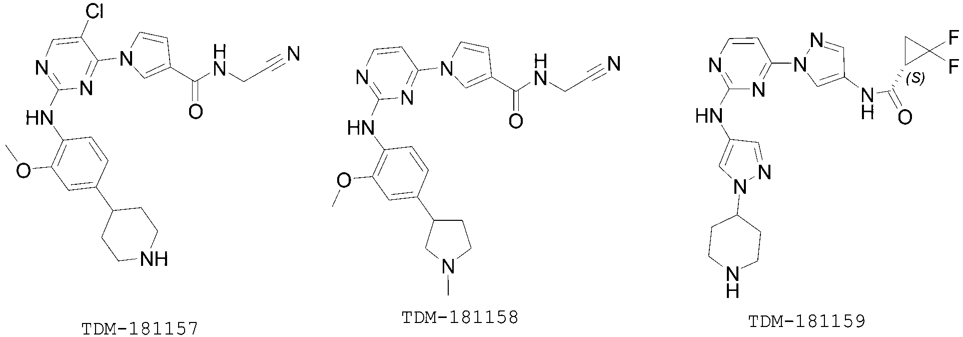

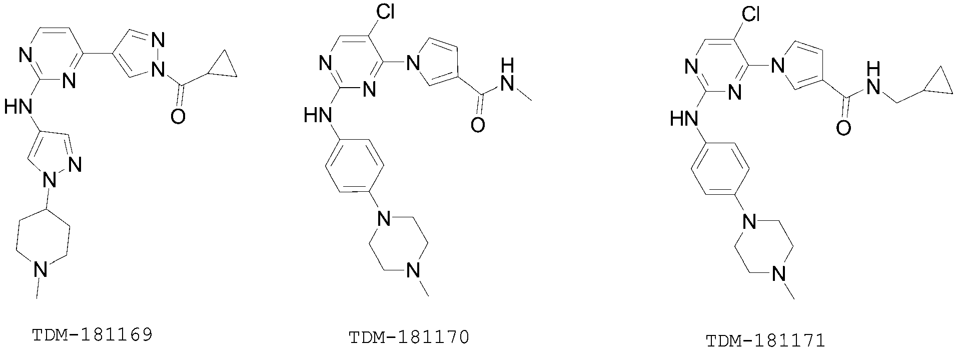

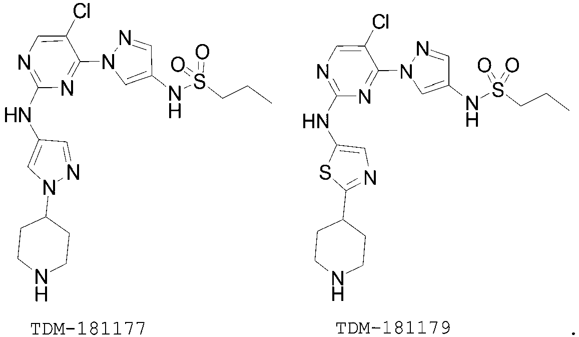

In one set of embodiments, the pharmaceutically acceptable salt is a formate salt.

In one set of embodiments, the isotopic derivative is a deuterated compound.

Another aspect of the present application further provides a method for preparing the pyrimidinamine-based compound, including: preparing the compound of Formula I from a compound of Formula II and a compound of Formula III; or
when D is 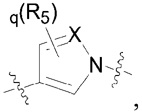 the compound of Formula I may alternatively be prepared in the following procedure: preparing a compound of Formula V from a compound of Formula IV and a compound of Formula II, and then preparing the compound of Formula I from the compound of Formula V;
optionally, the preparation process includes necessary protection and deprotection steps; 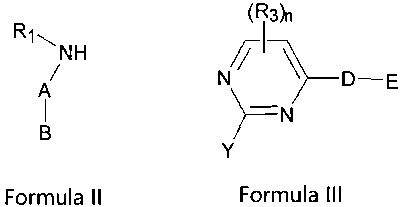 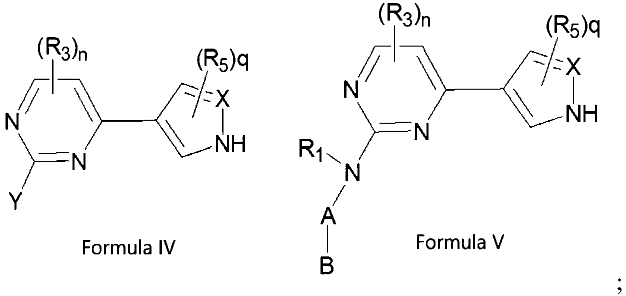
where Y is a leaving group, preferably halogen, more preferably Cl, and other groups are as defined above.

In one set of embodiments, the amino or imino group on ring B in Formula II is first protected; the protected compound is then coupled with a compound of Formula III, followed by removing the protecting group to obtain the compound of Formula I.

In one set of embodiments, when D is 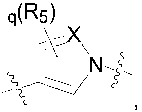 the imino group on ring D in the compound of Formula IV is first protected, and the amino group or the imino group on ring B in Formula II is optionally protected. The protected compound of Formula IV is then coupled with the optionally protected compound of Formula II, followed by removing the substituent on ring D to obtain the compound of Formula V. Finally, the compound of Formula I is obtained via a coupling reaction and optional removal of the protecting group on ring B.

The present application provides a pharmaceutical composition, in which the pyrimidinamine-based compound according to the present application is used as an active ingredient, and the pharmaceutical composition further contains a pharmaceutically acceptable carrier or excipient.

The present application provides the use of the pyrimidinamine-based compound in the preparation of a NUAK1 or NUAK2 inhibitor.

The present application provides the use of the pyrimidinamine-based compound in preparation of a medicament, wherein the compound is useful for the prevention or treatment of the following diseases by inhibiting NUAK1 or NUAK2: neuropsychiatric diseases, metabolic diseases, tumors, visceral fibrotic diseases, or skin fibrotic diseases.

In one set of embodiments, the disease is Parkinson's disease, Alzheimer's disease, diabetes, hyperlipidemia, obesity, liver cancer, leukemia, lymphoma, tumor metastasis, liver cirrhosis, renal fibrosis, pulmonary fibrosis, post-myocarditis sequelae, scleroderma, keloids, or hypertrophic scar; or the compound is used solely for alleviating a traumatic or postoperative scar.

### Beneficial effects of the present disclosure:

The present disclosure provides a class of pyrimidinamine-based compounds, and *in vitro* kinase activity inhibition assays show that the compounds according to the present disclosure have excellent inhibitory activity against NUAK1/NUAK2 kinases. The compounds are useful for the prevention or treatment of the following diseases by inhibiting NUAK1 or NUAK2: neuropsychiatric diseases, metabolic diseases, tumors, visceral fibrotic diseases, or skin fibrotic diseases.

### DETAILED DESCRIPTION

The present disclosure is described in detail hereinafter. It should be understood that the specific embodiments described herein are intended merely to illustrate and explain the present disclosure, and are not intended to limit the present disclosure.

The endpoints and any values of the ranges disclosed herein are not limited to those precise ranges or values, and such ranges or values should be understood to include values approximating those ranges or values. For numerical ranges, combinations may be made between the endpoint values of each range, between the endpoint values of each range and individual point values, and between individual point values to obtain one or more new numerical ranges, and such numerical ranges are to be regarded as specifically disclosed herein.

Before describing the present disclosure in detail, it should be understood that the terms used herein are for the purpose of describing particular embodiments only and are not intended to limit the scope of the present disclosure, which is to be limited solely by the appended claims. For a more complete understanding of the present disclosure described herein, the following terms are employed, with definitions set forth below. Unless otherwise defined, all technical and scientific terms used herein have the same meaning as commonly understood by one of ordinary skill in the art to which this disclosure belongs.

### Definitions

Unless otherwise specified, the following terms as used in the present disclosure have the definitions set forth below.

Features illustrated or described as part of one embodiment or group of embodiments may be used in another embodiment or group of embodiments to produce further embodiments.

In the present disclosure, 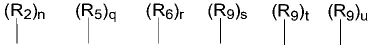 connected to a cyclic moiety indicate that n R₂ groups, q R₅ groups, r R₆ groups, s R₉ groups, t R₉ groups, and u R₉ groups may be attached at any possible position on said cyclic moiety.

In the present disclosure, 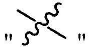 in some substituents represents the point of attachment. When D is 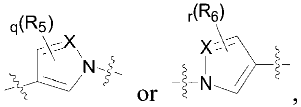 the left side of D is attached to the pyrimidine ring, and the right side of D is attached to the E moiety.

Unless otherwise specified, the term "optionally substituted" means that the hydrogen atoms on the substituted group are unsubstituted, or one or more substitutable positions on the substituted group are each independently substituted with substituent(s) each independently selected from the group consisting of deuterium, halogen, an amino group, a hydroxyl group, a nitro group, a cyano group, a sulfhydryl group, oxo, an optionally substituted C1-C8 alkyl group, an optionally substituted C1-C8 alkoxy group, an optionally substituted C1-C8 alkylthio group, and an optionally substituted C1-C8 alkylamino group. The substituent on the C1-C8 alkyl group is one or more selected from the group consisting of deuterium, halogen, an amino group, a hydroxyl group, a nitro group, a cyano group, and a sulfhydryl group. When the substituent is "oxo", it means that two hydrogen atoms at the same substitution position are replaced by an oxygen atom.

The term "heteroaryl group" refers to an aromatic monocyclic or polycyclic ring system having from 5 to 10 ring atoms, preferably from 5 to 8 ring atoms, more preferably from 5 to 6 ring atoms, where one, two, three, four or more ring atoms are heteroatoms and the remaining ring atoms are carbon atoms, where the heteroatoms are each independently selected from O, N, or S, and the number of heteroatoms is preferably 1, 2, 3, or 4. The heteroaryl may be a 5-membered aromatic monocyclic ring containing 1 or 2 heteroatoms selected from N or S, or a 5-membered aromatic monocyclic ring containing 1 or 2 N atoms. Examples of heteroaryl groups include, but are not limited to, a furanyl group, a thienyl group, an oxazolyl group, a thiazolyl group, an isoxazolyl group, an oxadiazolyl group, a thiadiazolyl group, a pyrrolyl group, a pyrazolyl group, an imidazolyl group, a triazolyl group, a tetrazolyl group, a pyridinyl group, a pyrimidinyl group, a pyrazinyl group, a pyridazinyl group, a thiodiazolyl group, a triazinyl group, a phthalazinyl group, a quinolinyl group, an isoquinolinyl group, a pteridinyl group, a purinyl group, an indolyl group, an isoindolyl group, an indazolyl group, a benzofuranyl group, a benzothienyl group, a benzopyridinyl group, a benzopyrimidinyl group, a benzopyrazinyl group, a benzimidazolyl group, a benzophthalazinyl group, a pyrrolo[2,3-b]pyridinyl group, an imidazo[1,2-a]pyridinyl group, a pyrazolo[1,5-a]pyridinyl group, a pyrazolo[1,5-a]pyrimidinyl group, an imidazo[1,2-b]pyridazinyl group, a [1,2,4]triazolo[4,3-b]pyridazinyl group, a [1,2,4]triazolo[1,5-a]pyrimidinyl group and a [1,2,4]triazolo[1,5-a]pyridinyl group.

The term "cycloalkyl group" refers to a saturated monocyclic, bicyclic, or tricyclic hydrocarbon system containing from 3 to 10 carbon atoms, wherein the monocyclic, bicyclic, or tricyclic system contains no aromatic ring. Bicyclic groups include bridged rings, spiro rings, fused rings, and the like. Preferably, the cycloalkyl group contains from 3 to 10 carbon atoms (C3-10 cycloalkyl), more preferably from 3 to 8 carbon atoms (C3-8 cycloalkyl), from 3 to 6 carbon atoms (C3-6 cycloalkyl), from 4 to 6 carbon atoms (C4-6 cycloalkyl), or from 5 to 6 carbon atoms (C5-6 cycloalkyl). Examples include, but are not limited to, a cyclopropyl group, a cyclobutyl group, a cyclopentyl group, a cyclohexyl group, a methylcyclopropyl group, a 2-ethyl-cyclopentyl group, and a dimethylcyclobutyl group.

The term "heterocycloalkyl group" refers to a saturated monocyclic, bicyclic, or polycyclic hydrocarbon group, preferably having from 3 to 10 ring atoms, where one, two, three or more ring atoms are selected from N, O, and S, and the remaining ring atoms are carbon atoms, including bridged rings, spiro rings, fused rings, and the like. Preferably, the heterocycloalkyl group has from 3 to 8 ring atoms (3- to 8-membered heterocycloalkyl), from 3 to 6 ring atoms (3- to 6-membered heterocycloalkyl), from 4 to 6 ring atoms (4- to 6-membered heterocycloalkyl), or from 5 to 6 ring atoms (5- to 6-membered heterocycloalkyl). The number of heteroatoms is preferably from 1 to 4, more preferably from 1 to 3 (i.e., 1, 2, or 3). The heterocycloalkyl may be a 5- to 6-membered monocyclic heterocycloalkyl containing 1 or 2 N atoms. Examples of heterocycloalkyl groups include a pyrrolidinyl group, an imidazolidinyl group, a tetrahydrofuranyl group, a piperidinyl group, a piperazinyl group, a pyranyl group, an aziridinyl group, an oxiranyl group, a thiiranyl group, an azetidinyl group, an oxetanyl group, a thietanyl group, oxacyclohexyl, a morpholinyl group, a thiomorpholinyl group, a dioxanyl group, a dithianyl group, an oxazolidinyl group, a thiazolidinyl group, a pyrazolidinyl group, an imidazolinidinyl group, and the like.

The term "alkyl group" refers to a monovalent saturated aliphatic hydrocarbon group, preferably a straight or branched chain group containing from 1 to 8 carbon atoms (C1-8 alkyl; the number of carbon atoms is 1, 2, 3, 4, 5, 6, 7, or 8), more preferably from 1 to 6 carbon atoms (C1-6 alkyl; the number of carbon atoms is 1, 2, 3, 4, 5, or 6). Examples include, but are not limited to, a methyl group, an ethyl group, an n-propyl group, an isopropyl group, an n-butyl group, an isobutyl group, a tert-butyl group, a sec-butyl group, an n-pentyl group, a neopentyl group, a 1,1-dimethylpropyl group, a 1,2-dimethylpropyl group, a 2,2-dimethylpropyl group, a 1-ethylpropyl group, a 2-methylbutyl group, a 3-methylbutyl group, an n-hexyl group, an n-heptyl group, and an n-octyl group.

The terms "alkoxy group", "alkylthio group", and "alkylamino group" refer to -O-alkyl group, -S-alkyl group, -NH-alkyl group or dialkylamino group, respectively, where the alkyl group is as defined above. Representative examples include, but are not limited to, a methoxy group, an ethoxy group, a propoxy group, an isopropoxy group, a butoxy group, a 1-methylpropoxy group, a 2-methylpropoxy group, a tert-butoxy group, a methylthio group, an ethylthio group, a propylthio group, an isopropylthio group, a butylthio group, a 1-methylpropylthio group, a 2-methylpropylthio group, a tert-butylthio group, a methylamino group, an ethylamino group, a propylamino group, a dimethylamino group, a diethylamino group, a dipropylamino group, and a methylethylamino group.

The term "halogen" refers to F, Cl, Br, and I.

The active compound of the present disclosure is understood to include the compound itself, as well as its stereoisomers, tautomers, isotopic derivatives, hydrates, solvates, prodrugs, or pharmaceutically acceptable salts thereof. The stereoisomers, tautomers, hydrates, solvates, prodrugs, and pharmaceutically acceptable salts of the compound may be prepared by conventional techniques in the art, and exert the same or similar effects *in vitro* and *in vivo* through substantially the same mechanism of action as said compound.

The term "stereoisomer" refers to isomers arising from the different spatial arrangement of atoms in a molecule, including configurational isomers and conformational isomers. Configurational isomers further include geometric isomers (or cis-trans isomers) and optical isomers (including enantiomers and diastereomers). Geometric isomers may exist in the present compounds. Optical isomers are substances having identical molecular structures, similar physicochemical properties, but different optical activities. The compounds according to the present disclosure may contain asymmetrically substituted carbon atoms in either the R or S configuration, wherein the terms "R" and "S" are as defined in IUPAC 1974 Recommendations for Section E, Fundamental Stereochemistry, Pure Appl. Chem. (1976) 45, 13-10. Compounds containing asymmetrically substituted carbon atoms (with equal amounts of R and S configurations) are racemic at those carbon atoms. An excess of one configuration relative to the other results in a higher proportion of that configuration, preferably an excess of about 85% to 90%, more preferably an excess of about 95% to 99%, even more preferably an excess of greater than about 99%. Accordingly, the present disclosure includes racemic mixtures, individual relative and absolute optical isomers, and mixtures of relative and absolute optical isomers.

The term "tautomer" refers to structural isomers having different energies that are interconvertible via a low energy barrier. Where tautomerization is possible (e.g., in solution), a chemical equilibrium of tautomers can be reached. For example, proton tautomers (also called prototropic tautomers) include interconversions via migration of a proton, such as ketoenol tautomerization and imine-enamine tautomerization. Valence tautomers include interconversions via reorganization of some bonding electrons.

The term "isotopic derivative" means that the compound of the present disclosure may exist in an isotopically labeled or enriched form, containing one or more atoms whose atomic weight or mass number differs from the atomic weight or mass number of the most abundant naturally occurring atom. Isotopes may be radioactive or non-radioactive. Isotopes of hydrogen, carbon, phosphorus, sulfur, fluorine, chlorine, and iodine include, but are not limited to, ²H, ³H, ¹³C, ¹⁴C, ¹⁵N, ¹⁸O, ³²P, ³⁵S, ¹⁸F, ³⁶Cl, and ¹²⁵I. Compounds containing other isotopes of these and/or other atoms are within the scope of the present disclosure. The isotopically labeled compounds of the present disclosure may be prepared by general methods known to those of ordinary skill in the art.

The term "hydrate" refers to an association formed by one or more water molecules and the compound of the present disclosure.

The term "solvate" refers to an association formed by one or more solvent molecules and the compound of the present disclosure.

The term "prodrug" refers to a derivative of an active drug designed to improve certain identified undesirable physical or biological properties. Physical properties typically relate to solubility (excessively high or insufficient lipid or aqueous solubility) or stability, while problematic biological properties include overly rapid metabolism or poor bioavailability, which may themselves be related to physicochemical properties.

The term "pharmaceutically acceptable salt" refers to a salt that is, within the scope of sound medical judgment, suitable for use in contact with the tissues of mammals, especially humans, without undue toxicity, irritation, allergic response, and the like, and commensurate with a reasonable benefit/risk ratio. When the compound is basic, pharmaceutically acceptable salts include salts formed from inorganic acids and salts formed from organic acids. When the compound is acidic, pharmaceutically acceptable salts include salts formed from inorganic bases and/or organic bases.

The term "pharmaceutically acceptable carrier" or "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, emulsifier, or the like that is approved by a relevant governmental regulatory authority for use in humans or livestock.

As used herein, the term "treatment" refers to any administration of a therapeutic agent according to a therapeutic regimen that achieves a desired effect, i.e., partial or complete reduction, amelioration, alleviation, inhibition, delay of onset, reduction in severity, and/or reduction in the incidence of one or more symptoms or features of a particular disease, disorder, and/or condition. In some embodiments, administration of a therapeutic agent according to a therapeutic regimen is associated with the achievement of the desired effect. Such treatment may be administered to a subject who does not exhibit signs of the relevant disease, disorder, and/or condition and/or to a subject who exhibits only early signs of the disease, disorder, and/or condition. Alternatively or additionally, such treatment may be administered to a subject who exhibits one or more established signs of the relevant disease, disorder, and/or condition. In some embodiments, treatment may be administered to a subject diagnosed with the relevant disease, disorder, and/or condition. In some embodiments, treatment may be administered to a subject known to have one or more predisposing factors that are statistically associated with an increased risk of developing the relevant disease, disorder, and/or condition.

According to the present disclosure, the medicament prepared for the pharmaceutical use described herein may contain, in addition to the pyrimidinamine-based compound of the present disclosure as an active ingredient, one or more other agents useful for the prevention or treatment of the relevant diseases as a further active ingredient. When the medicament contains multiple active ingredients, the active ingredients may be administered simultaneously, sequentially, or separately at the discretion of a physician.

In the following, the effects of specific compounds of the present disclosure are described in detail with reference to examples.

### Examples

### Example 1: General Method for Synthesizing Compound 534 (TDM-181134)

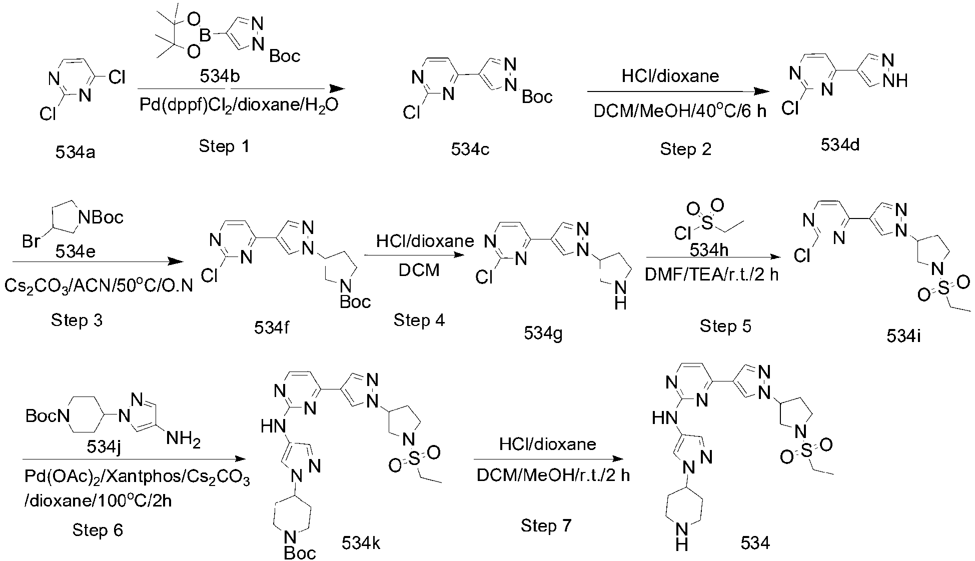

### Step 1: Compound 534c

### Tert-butyl 4-(2-chloropyrimidin-4-yl)-1H-pyrazole-1-carboxylate

Compound 534a (1 g, 6.7 mmol), compound 534b (1.97 g, 6.7 mmol), [1,1'-bis(diphenylphosphino)ferrocene]dichloropalladium(II) (490 mg, 0.67 mmol), cesium carbonate (4.4 g, 2 mmol), 1,4-dioxane (60 mL) and water (6 mL) were added to a 100 mL three-necked flask. The reaction mixture was purged with argon several times, heated to 70°C and stirred for one hour, and the reaction was complete as detected. Workup: The reaction mixture was added into ice water (200 mL), and the aqueous phase was extracted twice with ethyl acetate (3×100 mL). The organic phases were combined, washed with saturated brine, dried over anhydrous sodium sulfate, filtered with suction, and concentrated under reduced pressure. The resulting crude product was purified by column chromatography [eluent: (PE/EA) = 0-30%] to obtain the target compound as a yellow solid (compound 534c, 900 mg, yield 47.8%). LCMS [M+1]⁺ = 282.

### Step 2: Compound 534d

### 2-Chloro-4-(1H-pyrazol-4-yl)pyrimidine

Hydrogen chloride in 1,4-dioxane solution (8.9 mL, 35.6 mmol) was added to a solution of compound 534c (1 g, 3.56 mmol) in dichloromethane (80 mL) and methanol (8 mL). The reaction mixture was heated to 40°C and stirred for 6 hours, and the reaction was complete as detected. Workup: The reaction mixture was directly concentrated under reduced pressure to obtain the target compound as a white solid (compound 534d, 850 mg, yield 68%). LCMS [M+1]⁺ = 181.

### Step 3: Compound 534f

### Tert-butyl 3-(4-(2-chloropyrimidin-4-yl)-1H-pyrazol-1-yl)pyrrolidine-1-carboxylate

Compound 534e (1.255 g, 5.02 mmol) and cesium carbonate (4.08 g, 12.54 mmol) were added to a solution of compound 534d (755.2 mg, 4.18 mmol) in acetonitrile (150 mL). The reaction mixture was heated to 50°C and stirred overnight, and the reaction was complete as detected. Workup: The reaction mixture was directly concentrated under reduced pressure, and water and ethyl acetate were added for extraction. The organic phases were combined, washed with saturated brine, dried over anhydrous sodium sulfate, filtered with suction, and concentrated under reduced pressure. The resulting crude product was purified by column chromatography [eluent: EA/PE = 0-50%] to obtain the target compound as a white solid (compound 534f, 136 mg, yield 9.3%). LCMS [M+1]⁺ = 350.

### Step 4: Compound 534g

### 2-Chloro-4-(1-(pyrrolidin-3-yl)-1H-pyrazol-4-yl)pyrimidine

Hydrogen chloride in 1,4-dioxane solution (1.5 mL, 0.86 mmol) was added to a solution of compound 534f (136 mg, 0.39 mmol) in dichloromethane (15 mL). The reaction mixture was stirred at room temperature for 2 hours, and the reaction was complete as detected. Workup: The reaction mixture was directly concentrated under reduced pressure to obtain the target compound as a white solid (compound 534g, 97 mg, yield 93%). LCMS [M+1]⁺ = 250.

### Step 5: Compound 534i

### 2-Chloro-4-(1-(1-(ethylsulfonyl)pyrrolidin-3-yl)-1H-pyrazol-4-yl)pyrimidine

Triethylamine (78.9 mg, 0.78 mmol) and compound 534h (75.2 mg, 0.59 mmol) were added to a solution of compound 534g (97.38 mg, 0.39 mmol) in N,N-dimethylformamide (6 mL). The reaction mixture was stirred at room temperature for 2 hours, and the reaction was complete as detected. Workup: The reaction mixture was added into water, and the aqueous phase was extracted with ethyl acetate (3×50 mL). The organic phases were combined, washed with saturated brine, dried over anhydrous sodium sulfate, filtered with suction, and concentrated under reduced pressure. The resulting crude product was purified by column chromatography [eluent: EA/PE = 0~50%] to obtain the target compound as a white solid (compound 534i, 70 mg, yield 52.5%). LCMS [M+1]⁺ = 342.

### Step 6: Compound 534k

### Tert-butyl 4-(4-((4-(1-(ethylsulfonyl)pyrrolidin-3-yl)-1H-pyrazol-4-yl)pyrimidin-2-yl)amino)-1H-pyrazol-1-yl)piperidine-1-carboxylate

Compound 534i (48.5 mg, 0.14 mmol), compound 534j (45.3 mg, 0.17 mmol), palladium acetate (6.37 mg, 0.03 mmol), 4,5-bis(diphenylphosphino)-9,9-dimethylxanthene (32.84 mg, 0.06 mmol), cesium carbonate (92.46 mg, 0.28 mmol) and 1,4-dioxane (8 mL) were added to a 100 mL three-necked flask. The reaction mixture was purged with argon several times, heated to 100°C and stirred for 2 hours, and the reaction was complete as detected. Workup: The reaction mixture was concentrated under reduced pressure, and ethyl acetate (100 mL) and water (100 mL) were added for extraction. The organic phases were combined, washed with saturated brine, dried over anhydrous sodium sulfate, filtered with suction, and concentrated under reduced pressure. The resulting crude product was purified by column chromatography [eluent: (dichloromethane:methanol = 10:1)/DCM = 0-50%] to obtain the target compound as a white solid (compound 534k, 59 mg, yield 50.5%). LCMS [M+1]⁺ = 573.

### Step 7: Compound 534

### 4-(1-(1-(Ethylsulfonyl)pyrrolidin-3-yl)-1H-pyrazol-4-yl)-N-(1-(piperidin-4-yl)-1H-pyrazol-4-yl)pyrimidin-2-amine

Hydrogen chloride in 1,4-dioxane solution (0.52 mL, 2.07 mmol) was added to a solution of compound 534k (59.1 mg, 0.1 mmol) in dichloromethane (5 mL) and methanol (1 mL). The reaction mixture was stirred at room temperature for two hours, and the reaction was complete as detected. Workup: The reaction mixture was concentrated under reduced pressure, and the crude product was purified by preparative chromatography to obtain the target compound as a white solid (compound 534, 7.7 mg, yield 5.5%). LCMS [M+1]⁺ = 472.3.

¹H NMR (400 MHz, DMSO) δ 9.37 (s, 1H), 8.53 (s, 1H), 8.35 (d, J = 5.0 Hz, 2H), 8.16 (s, 1H), 7.97 (s, 1H), 7.57 (s, 1H), 7.00 (d, J = 5.1 Hz, 1H), 5.20-5.09 (m, 1H), 4.31 (s, 1H), 3.84-3.79 (m, 2H), 3.63 (d, J = 6.3 Hz, 1H), 3.53 (d, J = 5.6 Hz, 2H), 3.21-3.11 (m, 4H), 2.77 (t, J = 11.9 Hz, 2H), 2.47-2.38 (m, 2H), 2.03 (d, J = 11.2 Hz, 2H), 1.92 (d, J = 11.1 Hz, 2H), 1.22 (t, J = 7.4 Hz, 3H).

### Example 2: General Method for Synthesizing Compound 535 (TDM-181135)

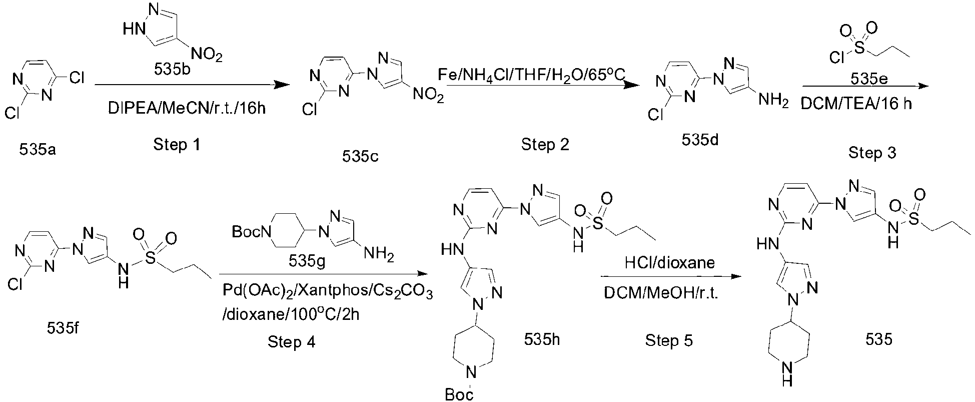

### Step 1: Compound 535c

### 2-Chloro-4-nitro-pyrazolopyrimidine

N,N-diisopropylethylamine (1.91 g, 0.01 mol) was added to a solution of compound 535a (2 g, 0.013 mol) and compound 535b (1.44 g, 0.013 mol) in acetonitrile (24 mL). The reaction mixture was stirred at room temperature for 16 hours. Upon completion, the mixture was concentrated under reduced pressure. Water (60 mL) was added to the residue, and the mixture was extracted with ethyl acetate (3×30 mL). The organic layers were combined, washed with saturated brine, and dried over sodium sulfate. The filtrate was concentrated under reduced pressure, and the residue was purified by silica gel chromatography (petroleum ether/ethyl acetate = 0-24/76) to obtain compound 535c as a white solid (713.3 mg, yield 23.6%). LCMS [M+1]⁺ = 226.0.

### Step 2: Compound 535d

### 2-Chloropyrimidin-4-yl-pyrazol-4-amine

Water (7.4 mL) was added to a solution of compound 535c (50 mg, 0.222 mmol), ammonium chloride (59.27 mg, 1.11 mmol) and iron powder (61.89 mg, 1.108 mmol) in tetrahydrofuran (7.4 mL). The reaction mixture was stirred at 65°C for 16 hours. Upon completion, the mixture was concentrated under reduced pressure. Water (60 mL) was added to the residue, and the mixture was extracted with ethyl acetate (3×30 mL). The organic layers were combined, washed with saturated brine, and dried over sodium sulfate. The filtrate was concentrated under reduced pressure, and the residue was purified by silica gel column chromatography (ethyl acetate/petroleum ether = 0-38/62) to obtain compound 535d as a yellow solid (45 mg, crude product). LCMS [M+1]⁺ = 196.0.

### Step 3: Compound 535f

### 1-(2-Chloropyrimidin-4-yl)-4-pyrazolylpropanesulfonamide

Triethylamine (67.29 mg, 0.67 mmol) and compound 535e (63.17 mg, 0.443 mmol) were added to a solution of compound 535d (43.36 mg, 0.222 mmol) in dichloromethane. The mixture was stirred at 40°C for 16 hours. Upon completion, the mixture was concentrated under reduced pressure. Water (30 mL) was added to the residue, and the mixture was extracted with ethyl acetate (3×40 mL). The organic layers were combined, washed with brine (50 mL), and dried over sodium sulfate. The filtrate was concentrated under reduced pressure, and the residue was purified by silica gel column chromatography (dichloromethane/dichloromethane containing 10% methanol = 0-44/56) to obtain compound 535f as a white solid (42.9 mg, yield 64%). LCMS [M+1]⁺ = 302.1.

### Step 4: Compound 535h

### Tert-butyl 4-(4-(4-(propylsulfonamido)-1H-pyrazol-1-ylpyrimidin-2-ylamino)-1H-pyrazol-1-yl)piperidine-1-carboxylate

4,5-bis(diphenylphosphino)-9,9-dimethylxanthene (40.5 mg, 0.07 mmol), palladium acetate (6.74 mg, 0.03 mmol) and cesium carbonate (107.52 mg, 0.33 mmol) were added to a solution of compound 535f (50 mg, 0.166 mol) and compound 535g (53.05 mg, 0.2 mmol) in hydrogen chloride in 1,4-dioxane (3 mL). The mixture was degassed under vacuum and purged with argon several times, then the reaction mixture was stirred at 100°C for 2 hours. Upon completion, the mixture was concentrated under reduced pressure, and the residue was purified by silica gel column chromatography (dichloromethane/dichloromethane containing 10% methanol = 0-60/40) to obtain compound 535h as a yellow solid (64.9 mg, yield 73.5%). LCMS [M+1]⁺ = 532.2.

### Step 5: Compound 535

### 2-(1-(piperidin-4-yl)-1H-pyrazol-4-yl)aminopyrimidin-4-yl)-1H-pyrazol-4-ylpropanesulfonamide

Hydrogen chloride in 1,4-dioxane (0.61 mL) was added to a solution of compound 535h (64.90 mg, 0.122 mmol) in solvent A (dichloromethane/methanol = 10:1). The reaction mixture was stirred at room temperature for 2 hours. Upon completion, the mixture was concentrated under reduced pressure, and the residue was purified by preparative chromatography (formic acid) to obtain compound 535 as a white solid (19.8 mg, yield 37.6%). LCMS [M+1]⁺ = 532.2.

¹H NMR (400 MHz, DMSO) δ 9.73 (s, 1H), 8.48 (d, J = 4.6 Hz, 1H), 8.35 (d, J = 5.8 Hz, 2H), 7.95 (s, 1H), 7.73 (s, 1H), 7.56 (s, 1H), 7.12 (d, J = 5.4 Hz, 1H), 4.36-4.27 (m, 2H), 3.22 (d, J = 12.6 Hz, 3H), 3.11-3.06 (m, 2H), 2.82 (t, J = 11.8 Hz, 3H), 2.06 (s, 2H), 1.98 (s, 2H), 1.70 (dd, J = 15.1, 7.5 Hz, 2H), 0.96 (t, J = 7.4 Hz, 4H).

The following compounds were synthesized in a similar manner to Example 2: 2-(2-Methoxy-4-(4-methylpiperazin-1-ylphenyl)aminopyrimidin-4-yl)-1H-pyrazol-4-ylpropanesulfonamide (TDM-181136), a yellow solid (16.9 mg, yield 21%); and 2-(2-(2-methoxy-4-piperidinylphenyl)aminopyrimidin-4-yl)-4-ylpyrazolylpropane-1-sulfonamide (TDM-181138), a white solid (7.5 mg, yield 16.3%).

| **TDM NO.** | **Structure** | **LCMS** [M+1]⁺ | **¹H-NMR** |
|---|---|---|---|
| TDM-181136 | | 487.0 | ¹H NMR (400 MHz, MeOD) δ 8.42 (d, J = 6.1 Hz, 2H), 8.39 (d, J = 5.5 Hz, 1H), 7.97 (d, J = 8.7 Hz, 1H), 7.69 (d, J = 0.6 Hz, 1H), 7.22 (d, J = 5.5 Hz, 1H), 6.72 (d, J = 2.5 Hz, 1H), 6.62 (dd, J = 8.8, 2.6 Hz, 1H), 3.90 (s, 3H), 3.37-3.33 (m, 4H), 3.12-3.09 (m, 4H), 3.07 (dd, J = 5.9, 3.7 Hz, 2H), 2.71 (s, 3H), 1.86-1.79 (m, 2H), 1.04 (t, J = 7.5 Hz, 3H). |
| TDM-181138 | | 472.2 | ¹H NMR (400, MHz, DMSO) δ 8.46 (d, J = 5.4 Hz, 2H), 8.40 (s, 1H), 8.30 (s, 1H), 7.84 (d, J = 8.2 Hz, 1H), 7.72 (d, J = 0.7 Hz, 1H), 7.17 (d, J = 5.4 Hz, 1H), 6.93 (d, J = 1.6 Hz, 1H), 6.81 (dd, J = 8.2, 1.6 Hz, 1H), 3.84 (s, 3H), 3.24 (s, 2H), 3.08 - 3.04 (m, 2H), 2.84 (dd, J = 12.6, 10.1 Hz, 2H), 2.74 (dd, J = 16.1, 7.7 Hz, 1H), 1.85 (s, 2H), 1.71 (td, J = 15.6, 8.1 Hz, 4H), 0.96 (t, J = 7.4 Hz, 3H). |

### Example 3: General Method for Synthesizing Compound 539 (TDM-181139)

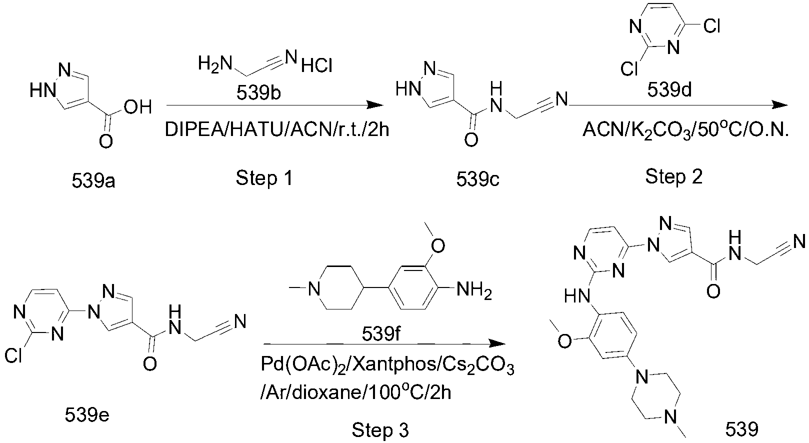

### Step 1: Compound 539c

### N-(Cyanomethyl)-1H-pyrazole-4-carboxamide

N,N-diisopropylethylamine (472 mg, 3.65 mmol) was added to a solution of compound 539b (124 mg, 1.338 mmol) in acetonitrile (5 mL). The mixture was stirred, then 2-(7-azabenzotriazol-1-yl)-N,N,N',N'-tetramethyluronium hexafluorophosphate (407 mg, 1.07 mmol) and compound 539a (100 mg, 0.892 mmol) were added. The mixture was stirred at room temperature for 2 hours. The mixture was concentrated under reduced pressure, and the residue was purified by silica gel chromatography (dichloromethane/10% NH₃-methanol in dichloromethane = 0-80/20) to obtain compound 539c as a yellow oil (402 mg, crude product). LCMS [M+1]⁺ = 151.

### Step 2: Compound 539e

### 1-(2-Chloropyrimidin-4-yl)-N-(cyanomethyl)-1H-pyrazole-4-carboxamide

Potassium carbonate (370 mg, 2.676 mmol) was added to a solution of compound 539d (133 mg, 0.892 mmol) and compound 539c (crude product) in acetonitrile (10 mL). The mixture was heated to 50°C and stirred overnight. The mixture was added into water (100 mL) and extracted with ethyl acetate (3×40 mL). The organic layers were combined, washed with brine, dried over sodium sulfate, and the filtrate was concentrated under reduced pressure. The residue was purified by silica gel chromatography (ethyl acetate/petroleum ether = 0-40/60) to obtain compound 539e as a white solid (91 mg, yield 38.8%). LCMS [M+1]⁺ = 249.

¹H NMR (400 MHz, DMSO) δ 9.28-9.22 (m, 2H), 8.89 (d, J = 5.5 Hz, 1H), 8.34 (s, 1H), 7.98 (d, J = 5.5 Hz, 1H), 4.34 (d, J = 5.5 Hz, 2H).

### Step 3: Compound 539

### N-(Cyanomethyl)-1-(2-((2-methoxy-4-(4-methylpiperazin-1-yl)phenyl)amino)pyrimidin-4-yl)-1H-pyrazole-4-carboxamide

1,4-Dioxane (5 mL) was added to a mixture of compound 539e (45 mg, 0.171 mmol), compound 539f (45.4 mg, 0.206 mmol), palladium acetate (7.7 mg, 0.034 mmol), xantphos (40 mg, 0.068 mmol) and cesium carbonate (111 mg, 0.342 mmol). The mixture was degassed under vacuum, purged with argon several times, heated to 100°C and stirred for 2 hours. The mixture was concentrated under reduced pressure, and the residue was purified by silica gel chromatography (dichloromethane/ 10% methanol in dichloromethane = 0-40/60) and preparative HPLC (mobile phase containing 0.5% formic acid) to obtain compound 539 (TDM-181139) as a brown solid (7.6 mg, yield 9%). LCMS [M+1]⁺ = 448.

¹H NMR (400 MHz, DMSO) δ 9.25 (t, J = 5.4 Hz, 1H), 8.99 (s, 1H), 8.50 (d, J = 5.3 Hz, 1H), 8.35 (s, 1H), 8.26 (s, 1H), 8.15 (s, 1H), 7.66 (t, J = 8.9 Hz, 1H), 7.19 (d, J = 5.3 Hz, 1H), 6.66 (d, J = 2.3 Hz, 1H), 6.54 (dd, J = 8.7, 2.3 Hz, 1H), 4.37-4.28 (m, 2H), 3.81 (s, 3H), 3.20-3.14 (m, 4H), 2.53 (s, 4H), 2.27 (s, 3H).

### Example 4 General Method for Synthesizing Compound 540 (TDM-181140)

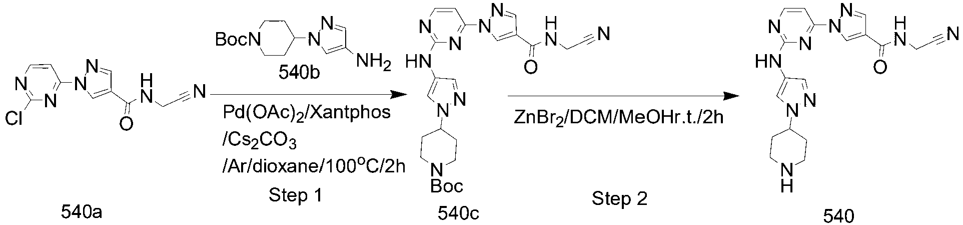

### Step 1: Compound 540c

### Tert-butyl 4-(4-((4-(4-(cyanomethyl)carbamoyl)-1H-pyrazol-1-yl)pyrimidin-2-yl)amino)-1H-pyrazol-1-yl)piperidine-1-carboxylate

1,4-Dioxane (8 mL) was added to a mixture of compound 540a (100 mg, 0.381 mmol), compound 540b (122 mg, 0.457 mmol), palladium acetate (17 mg, 0.076 mmol), xantphos (88 mg, 0.152 mmol) and cesium carbonate (248 mg, 0.762 mmol). The mixture was degassed under vacuum, purged with argon several times, heated to 100°C and stirred for 2 hours. The mixture was concentrated under reduced pressure, and the residue was purified by silica gel chromatography (dichloromethane/10% methanol in dichloromethane = 0~45/55) to obtain compound 540c as a yellow solid (146 mg, yield 77.8%). LCMS [M+1-100]⁺ = 393.

### Step 2: Compound 540

### N-(Cyanomethyl)-1-(2-((1-(piperidin-4-yl)-1H-pyrazol-4-yl)amino)pyrimidin-4-yl)-1H-pyrazole-4-carboxamide

Zinc bromide (220 mg, 0.976 mmol) was added to a solution of compound 540c (60 mg, 0.122 mmol) in dichloromethane (5 mL) and methanol (1 mL) under argon atmosphere. The mixture was stirred at room temperature for 2 hours. The mixture was concentrated under reduced pressure, and the residue was purified by preparative HPLC (formic acid) to obtain compound 540 (TDM-181140) as a yellow solid (17.4 mg, yield 36.3%). LCMS [M+1]⁺ = 393.

¹H NMR (400 MHz, MeOD) δ 9.09 (s, 1H), 8.50 (d, J = 5.3 Hz, 1H), 8.17 (s, 1H), 8.12 (s, 1H), 7.67 (s, 1H), 7.28 (d, J = 5.4 Hz, 1H), 4.60 (s, 1H), 4.34 (s, 2H), 3.58 (d, J = 13.0 Hz, 2H), 3.28-3.17 (m, 2H), 2.41-2.20 (m, 4H).

### Example 5 General Method for Synthesizing Compound 545 (TDM-181145)

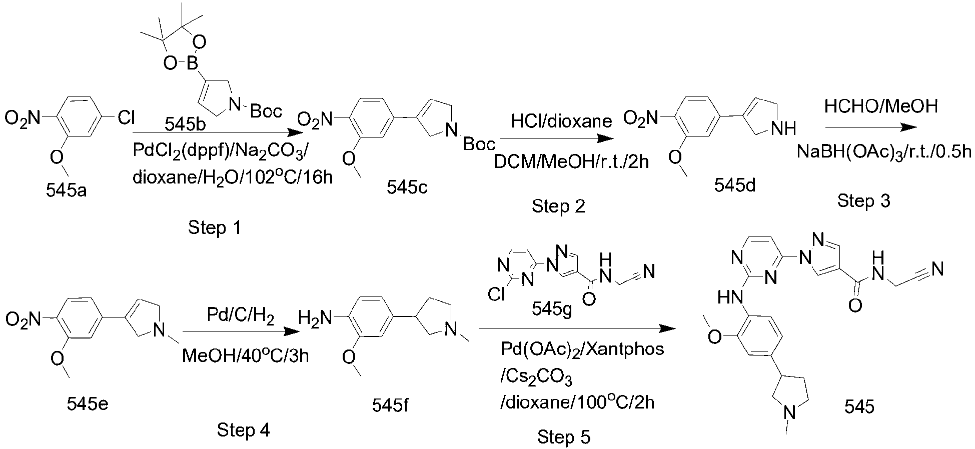

### Step 1: Compound 545c

### Tert-butyl 3-(3-methoxy-4-nitrophenyl)-2,5-dihydropyrrole-1-carboxylate

1,4-Dioxane (20 mL) and water (5 mL) were added to a mixture of compound 545a (534.6 mg, 2.85 mmol), compound 545b (842.0 mg, 2.85 mmol), [1,1'-bis(diphenylphosphino)ferrocene]dichloropalladium(II) (104.63 mg, 0.14 mmol) and sodium carbonate (694.76 mg, 6.56 mmol). The mixture was degassed under vacuum, purged with nitrogen, heated to 102°C and stirred for 16 hours. The mixture was concentrated under reduced pressure. Water (100 mL) was added to the residue, and the mixture was extracted with ethyl acetate (3×60 mL). The organic layers were combined, and the filtrate was concentrated under reduced pressure. The residue was purified by silica gel column chromatography (petroleum ether/ethyl acetate = 0-76/24) to obtain compound 545c as a yellow solid (478.3 mg, yield 52.4%). LCMS [M+1-56]⁺ = 265.1.

### Step 2: Compound 545d

### 3-Methoxy-4-nitrophenyl-2,5-dihydropyrrole

Hydrogen chloride in 1,4-dioxane (5.91 mL) was added to a solution of compound 545c (378.3 mg, 1.181 mmol) in solvent A (dichloromethane/methanol = 10:1, 15 mL). The reaction solution was stirred at room temperature for 2 hours. The mixture was concentrated under reduced pressure. Water (60 mL) was added to the residue, and the mixture was extracted with ethyl acetate (3×30 mL). The organic layers were combined, washed with brine (50 mL), dried over sodium sulfate, and the filtrate was concentrated under reduced pressure to obtain compound 545d as a brown solid (194.6 mg, yield 74.8%). LCMS [M+1]⁺ = 221.2.

### Step 3: Compound 545e

### 3-Methoxy-4-nitrophenyl-2,5-dihydropyrrole

Formaldehyde (214.86 mg) was added to a solution of compound 545d (194.6 mg, 0.884 mmol) in methanol (12 mL). The mixture was stirred at room temperature for 30 minutes, then sodium triacetoxyborohydride (749.1 mg, 3.53 mmol) was added, and stirring was continued for 10 minutes. The mixture was concentrated under reduced pressure, and the residue was purified by silica gel chromatography (petroleum ether/ethyl acetate = 0%-100%) to obtain compound 545e as a yellow solid (85.3 mg, yield 41.2%). LCMS [M+1]⁺ = 235.2.

### Step 4: Compound 545f

### 2-Methoxy-4-(1-methylpyrrolidin-3-yl)aniline

Palladium on carbon (20 mg) was added to a solution of compound 545e (85.3 mg, 0.36 mmol) in methanol (3 mL) at room temperature. The mixture was degassed under vacuum, purged with hydrogen, and stirred for 3 hours. The mixture was concentrated under reduced pressure, and the residue was purified by silica gel column chromatography (dichloromethane/10% methanol in dichloromethane = 0-77/23) to obtain compound 545f as a yellow solid (29 mg, yield 38.7%). The mixture was concentrated under reduced pressure, and the residue was used directly in the next step. LCMS [M+1]⁺ = 207.2.

### Step 5: Compound 545

### N-(Cyanomethyl)-1-(2-((2-methoxy-4-(1-methylpyrrolidin-3-yl)phenyl)amino)pyrimidin-4-yl)-1H-pyrazole-4-carboxamide

4,5-Bis(diphenylphosphino)-9,9-dimethylxanthene (32.4 mg, 0.06 mmol), palladium acetate (6.27 mg, 0.028 mmol) and cesium carbonate (91.23 mg, 0.28 mmol) were added to a solution of compound 545f (36.76 mg, 0.14 mmol) and compound 545g (29 mg, 0.14 mmol) in 1,4-dioxane (4 mL). The mixture was degassed under vacuum, purged with argon, and heated to 100°C with stirring for 2 hours. The mixture was concentrated under reduced pressure. Water (30 mL) was added to the residue, and the mixture was extracted with ethyl acetate (3×30 mL). The organic layers were combined, washed with brine (50 mL), dried over sodium sulfate, and the filtrate was concentrated under reduced pressure. The residue was purified by silica gel column chromatography (dichloromethane/10% methanol in dichloromethane = 0-100/0), then further purified by preparative HPLC (formic acid) to obtain compound 545 as a white solid (6.5 mg, yield 10.7%). LCMS [M+1]⁺ = 433.2.

¹H NMR (400 MHz, DMSO) δ 9.28 (s, 1H), 9.06 (s, 1H), 8.57 (d, J = 5.3 Hz, 1H), 8.39 (s, 1H), 8.26 (d, J = 11.4 Hz, 2H), 7.94 (d, J = 8.1 Hz, 1H), 7.27 (d, J = 5.3 Hz, 1H), 7.01 (s, 1H), 6.94 (d, J = 8.1 Hz, 1H), 4.34 (d, J = 5.2 Hz, 2H), 3.86 (s, 3H), 3.42-3.33 (m, 1H), 3.04 (t, J = 8.6 Hz, 1H), 2.80 (t, J = 6.9 Hz, 2H), 2.64 (t, J = 8.5 Hz, 1H), 2.43 (s, 3H), 2.36-2.22 (m, 1H), 1.87 (dd, J = 12.6, 7.6 Hz, 1H), 1.23 (s, 1H).

### Example 6 General Method for Synthesizing Compound 546 (TDM-181146)

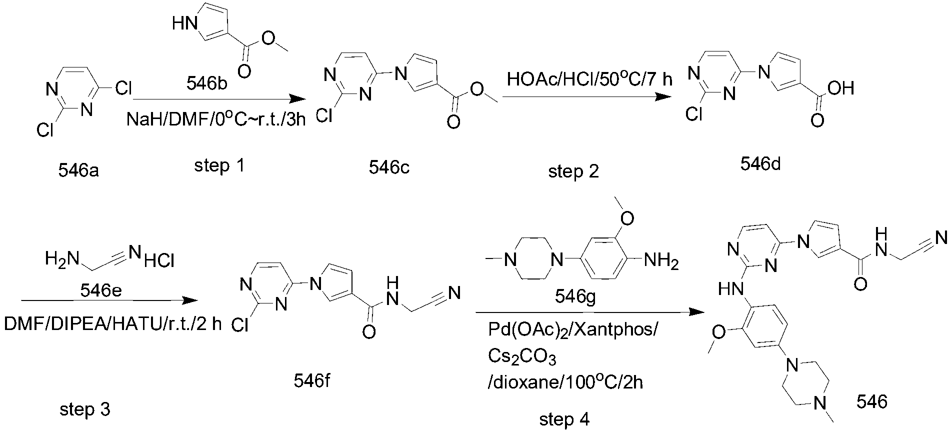

### Step 1: Compound 546c

### Methyl 1-(2-chloropyrimidin-4-yl)-1H-pyrrole-3-carboxylate

Sodium hydride (384 mg, 9.59 mmol) was added to a solution of compound 546b (1 g, 9.59 mmol) in N,N-dimethylformamide (20 mL) at 0°C. The reaction solution was warmed to room temperature and stirred for 30 minutes, then cooled back to 0°C. Compound 546a (1.43 g, 9.59 mmol) was added, and the solution was stirred at room temperature for 3 hours. The reaction was complete as detected. Workup: The reaction mixture was added into water and extracted with ethyl acetate (3×60 mL). The organic phases were combined, washed with saturated brine, dried over anhydrous sodium sulfate, filtered with suction, and concentrated under reduced pressure. The crude product was purified by column chromatography [eluent: (EA/PE) = 0-15%] to obtain compound 546c as a white solid (987 mg, yield 41%). LCMS [M+1]⁺ = 238.

¹H NMR (400 MHz, DMSO) δ 8.83 (d, J = 5.7 Hz, 1H), 8.39 (s, 1H), 8.08 (d, J = 5.7 Hz, 1H), 7.88-7.79 (m, 1H), 6.74 (dd, J = 3.1, 1.4 Hz, 1H), 3.79 (s, 3H).

### Step 2: Compound 546d

### 1-(2-Chloropyrimidin-4-yl)-1H-pyrrole-3-carboxylic acid

A solution of compound 546c (550 mg, 2.3 mmol) in acetic acid (17.5 mL) and concentrated hydrochloric acid (8.75 mL) was heated to 50°C and stirred for 7 hours. The reaction was complete as detected. Workup: The reaction mixture was added into water, and the aqueous phase was extracted with ethyl acetate (2×100 mL). The organic phases were combined, washed with saturated brine, dried over anhydrous sodium sulfate, filtered with suction, and concentrated under reduced pressure. The crude product was purified by column chromatography [eluent: (dichloromethane/methanol = 10:1)/dichloromethane = 0-80%] to obtain compound 546d as a white solid (95.8 mg, yield 18.6%). LCMS [M+1]+ = 224.

### Step 3: Compound 546f

### 1-(2-Chloropyrimidin-4-yl)-N-(cyanomethyl)-1H-pyrrole-3-carboxamide

2-(7-Azabenzotriazol-1-yl)-N,N,N',N'-tetramethyluronium hexafluorophosphate (163.1 mg, 0.43 mmol), N,N-diisopropylethylamine (92.4 mg, 0.72 mmol) and compound 546e (26.4 mg, 0.29 mmol) were added to a solution of compound 546d (64 mg, 0.29 mmol) in N,N-dimethylformamide (10 mL). The reaction solution was stirred at room temperature for 2 hours, and the reaction was complete as detected. Workup: The reaction mixture was added into water and extracted with ethyl acetate (3×100 mL). The organic phases were combined, washed with saturated brine, dried over anhydrous sodium sulfate, filtered with suction, and concentrated under reduced pressure. The crude product was purified by column chromatography [eluent: (dichloromethane/methanol = 10:1)/dichloromethane = 0-80%] to obtain compound 546f as a white solid (59.7 mg, yield 53.3%). LCMS [M+1]⁺ = 262.

### Step 4: Compound 546

### N-(Cyanomethyl)-1-(2-((2-methoxy-4-(4-methylpiperazin-1-yl)phenyl)amino)pyrimidin-4-yl)-1H-pyrrole-3-carboxamide

Compound 546f (30 mg, 0.12 mmol), compound 546g (30.5 mg, 0.14 mmol), palladium acetate (5.16 mg, 0.02 mmol), 4,5-bis(diphenylphosphino)-9,9-dimethylxanthene (26.6 mg, 0.05 mmol), cesium carbonate (75 mg, 0.23 mmol) and 1,4-dioxane (6 mL) were added to a 100 mL three-necked flask. The reaction mixture was purged with argon several times, heated to 100°C and stirred for 2 hours. The reaction was complete as detected. Workup: The reaction mixture was concentrated under reduced pressure. Ethyl acetate (100 mL) and water (100 mL) were added for extraction. The organic phases were combined, washed with saturated brine, dried over anhydrous sodium sulfate, and concentrated under reduced pressure. The crude product was purified by preparative chromatography to obtain compound 546 as a white solid (59 mg, yield 50.5%). LCMS [M+1]⁺ = 447.2.

¹H NMR (400 MHz, DMSO) δ 8.84 (t, J = 5.6 Hz, 1H), 8.42 (d, J = 5.5 Hz, 1H), 8.25 (s, 2H), 8.17 (s, 1H), 7.72-7.67 (m, 1H), 7.64 (d, J = 8.7 Hz, 1H), 7.10 (d, J = 5.5 Hz, 1H), 6.74 (dd, J = 3.2, 1.6 Hz, 1H), 6.65 (d, J = 2.4 Hz, 1H), 6.53 (dd, J = 8.8, 2.4 Hz, 1H), 4.27 (d, J = 5.5 Hz, 2H), 3.81 (s, 3H), 3.19-3.12 (m, 4H), 2.49 (d, J = 5.1 Hz, 4H), 2.25 (s, 3H).

N-(Cyanomethyl)-1-(2-((4-(4-methylpiperazin-1-yl)phenyl)amino)pyrimidin-4-yl)-1H-pyrrole-3-carboxamide (TDM-181147) was synthesized in a similar manner to Example 6 as a white solid (11.8 mg, yield 24.6%).

| **TDM NO.** | **Structure** | **LCMS** [M+1]⁺ | **¹H-NMR** |
|---|---|---|---|
| TDM-181147 | | 417.2 | ¹H NMR (400 MHz, DMSO) δ 9.55 (s, 1H), 8.85 (t, J = 5.6 Hz, 1H), 8.48 (d, J = 5.5 Hz, 1H), 8.28 (t, J = 1.9 Hz, 1H), 8.17 (s, 1H), 7.73 (dd, J = 3.2, 2.3 Hz, 1H), 7.56 (d, J = 9.1 Hz, 2H), 7.12 (d, J = 5.5 Hz, 1H), 6.93 (d, J = 9.1 Hz, 2H), 6.77 (dd, J = 3.2, 1.7 Hz, 1H), 4.28 (d, J = 5.6 Hz, 3H), 3.11-3.07 (m, 4H), 2.49 (d, J = 4.4 Hz, 4H), 2.25 (s, 3H). |

### Example 7 General Method for Synthesizing Compound 548 (TDM-181148)

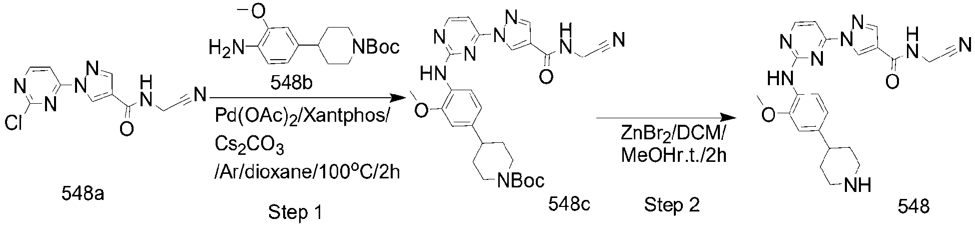

### Step 1: Compound 548c

### Tert-butyl 4-(4-((4-(4-(cyanomethyl)carbamoyl)-1H-pyrazol-1-yl)pyrimidin-2-yl)amino)-3-methoxyphenyl)piperidine-1-carboxylate

1,4-Dioxane (5 mL) was added to a mixture of compound 548a (70 mg, 0.266 mmol), compound 548b (98 mg, 0.319 mmol), palladium acetate (12 mg, 0.053 mmol), xantphos (61.6 mg, 0.106 mmol) and cesium carbonate (173 mg, 0.532 mmol). The mixture was degassed under vacuum, purged with argon several times, heated to 100°C and stirred for 2 hours. The mixture was concentrated under reduced pressure, and the residue was purified by silica gel chromatography (dichloromethane/10% methanol in dichloromethane = 0-14/86) to obtain compound 548c as a yellow solid (81 mg, yield 57.2%). LCMS [M+1]⁺ = 533.

### Step 2: Compound 548

### N-(Cyanomethyl)-1-(2-((2-methoxy-4-(piperidin-4-yl)phenyl)amino)pyrimidin-4-yl)-1H-pyrazole-4-carboxamide

Zinc bromide (247 mg, 1.096 mmol) was added to a solution of compound 548c (73 mg, 0.137 mmol) in dichloromethane (5 mL) and methanol (0.5 mL) under argon atmosphere. The mixture was stirred at room temperature for 2 hours. The mixture was concentrated under reduced pressure, and the residue was purified by preparative HPLC (formic acid) to obtain the desired product as a colorless oil (compound 548, TDM-181148, 6 mg, yield 10.1%). LCMS [M+1]⁺ = 433.

¹H NMR (400 MHz, DMSO) δ 9.46 (s, 1H), 9.08 (s, 1H), 8.57 (d, J = 5.4 Hz, 1H), 8.48 (s, 1H), 8.40 (s, 1H), 8.28 (d, J = 6.2 Hz, 1H), 7.98 (d, J = 8.2 Hz, 1H), 7.28 (d, J = 5.4 Hz, 1H), 6.96 (s, 1H), 6.88 (d, J = 8.2 Hz, 1H), 4.34 (d, J = 4.9 Hz, 2H), 3.87 (s, 3H), 3.67 (s, 1H), 3.32 (d, J = 11.6 Hz, 2H), 2.92 (t, J = 11.5 Hz, 2H), 2.80 (t, J = 11.5 Hz, 1H), 1.98-1.76 (m, 4H).

### Example 8 General Method for Synthesizing Compound 552 (TDM-181152)

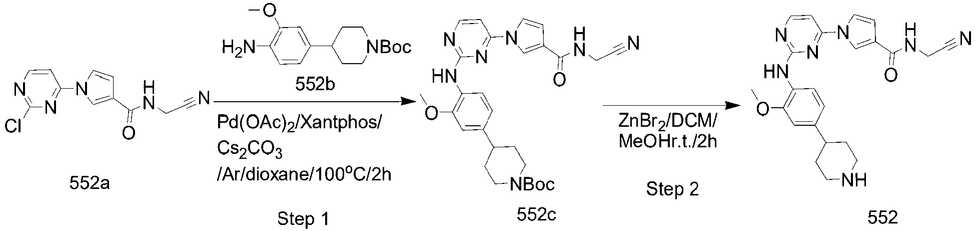

### Step 1: Compound 552c

### Tert-butyl 4-(4-((4-(3-(cyanomethyl)carbamoyl)-1H-pyrrol-1-yl)pyrimidin-2-yl)amino)-3-methoxyphenyl)piperidine-1-carboxylate

1,4-Dioxane (5 mL) was added to a mixture of compound 552a (80 mg, 0.306 mmol), compound 552b (112 mg, 0.367 mmol), palladium acetate (14 mg, 0.061 mmol), xantphos (70.8 mg, 0.112 mmol) and cesium carbonate (199 mg, 0.612 mmol). The mixture was degassed under vacuum, purged with argon several times, heated to 100°C and stirred for 2 hours. The mixture was concentrated under reduced pressure, and the residue was purified by silica gel chromatography (dichloromethane/10% methanol in dichloromethane = 0-55/45) to obtain compound 552c as a yellow solid (96 mg, yield 59%). LCMS [M+1]⁺ = 532.

### Step 2: Compound 552

### N-(Cyanomethyl)-1-(2-((2-methoxy-4-(piperidin-4-yl)phenyl)amino)pyrimidin-4-yl)-1H-pyrrole-3-carboxamide

Zinc bromide (325 mg, 1.445 mmol) was added to a solution of compound 552c (96 mg, 0.181 mmol) in dichloromethane (10 mL) and methanol (1 mL) under argon atmosphere. The mixture was stirred at room temperature for 2 hours. The mixture was concentrated under reduced pressure, and the residue was purified by preparative HPLC (formic acid) to obtain compound 552 as a white solid (20 mg, yield 25.6%). LCMS [M+1]⁺ = 432.

¹H NMR (400 MHz, DMSO) δ 8.91 (t, J = 5.3 Hz, 1H), 8.49 (d, J = 5.5 Hz, 1H), 8.40 (s, 1H), 8.31 (d, J = 6.7 Hz, 2H), 7.94 (d, J = 8.2 Hz, 1H), 7.74 (s, 1H), 7.19 (d, J = 5.5 Hz, 1H), 6.93 (s, 1H), 6.85 (d, J = 8.1 Hz, 1H), 6.77 (s, 1H), 4.28 (d, J = 5.4 Hz, 2H), 3.86 (s, 3H), 3.66 (s, 1H), 3.28 (d, J = 11.7 Hz, 2H), 2.87 (t, J = 11.7 Hz, 2H), 2.77 (t, J = 11.8 Hz, 1H), 1.89 (d, J = 12.3 Hz, 2H), 1.78 (dd, J = 22.6, 12.1 Hz, 2H).

The following compounds were synthesized in a similar manner to Example 8: N-(Cyanomethyl)-1-(2-((1-(piperidin-4-yl)-1H-pyrazol-4-yl)amino)pyrimidin-4-yl)-1H-pyrrole-3-carboxamide (TDM-181153), an off-white solid (8.5 mg, yield 9%); and N-(cyanomethyl)-1-(2-((4-(piperidin-4-yl)phenyl)amino)pyrimidin-4-yl)-1H-pyrrole-3-carboxamide (TDM-181154), a white solid (25.2 mg, yield 28.6%).

| TDM NO. | Structure | LCMS [M+1]+ | 1H-NMR |
|---|---|---|---|
| TDM-181153 | | 392.1 | ¹H NMR (400 MHz, DMSO) δ 9.68 (s, 1H), 8.88 (s, 1H), 8.49 (d, J = 5.2 Hz, 1H), 8.28 (d, J = 13.5 Hz, 2H), 7.93 (s, 1H), 7.75 (s, 1H), 7.62 (s, 1H), 7.10 (d, J = 5.5 Hz, 1H), 6.78 (s, 1H), 4.40 (s, 1H), 4.29 (d, J = 5.5 Hz, 2H), 3.28 - 3.23 (m, 2H), 2.92 (s, 2H), 2.10 (s, 2H), 2.01 (d, J = 10.7 Hz, 2H). |
| TDM-181154 | | 402.47 | ¹H NMR (400 MHz, DMSO) δ 9.77 (s, 1H), 8.90 (d, J = 5.6 Hz, 1H), 8.53 (d, J = 5.5 Hz, 1H), 8.39 (s, 1H), 8.31 (t, J = 1.9 Hz, 1H), 7.76 (dd, J = 3.2, 2.3 Hz, 1H), 7.69 (d, J = 8.6 Hz, 2H), 7.23-7.15 (m, 3H), 6.79 (dd, J = 3.2, 1.7 Hz, 1H), 4.29 (d, J = 5.5 Hz, 2H), 3.27 (d, J = 12.4 Hz, 2H), 2.87 (dd, J = 12.6, 10.4 Hz, 2H), 2.73 (s, 1H), 1.86 (d, J = 12.7 Hz, 2H), 1.79-1.65 (m, 2H). |

### Example 9 General Method for Synthesizing Compound 555 (TDM-181155)

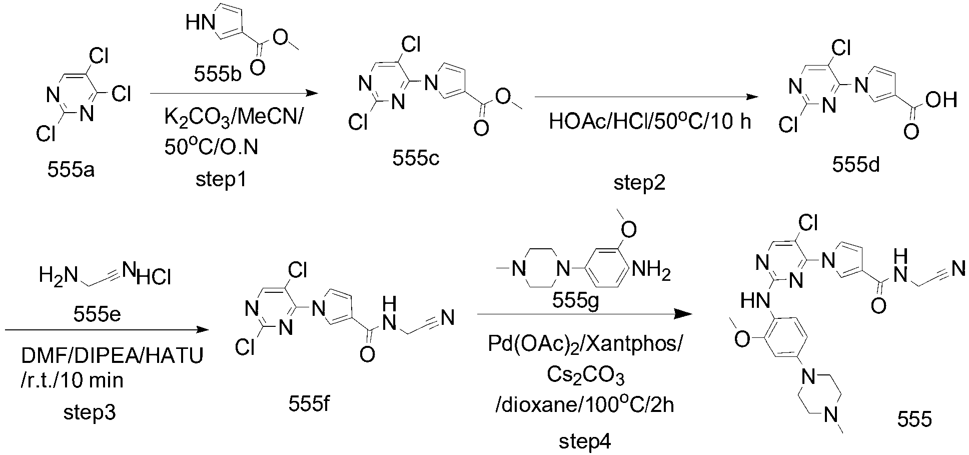

### Step 1: Compound 555c

### Methyl 1-(2,5-dichloropyrimidin-4-yl)-1H-pyrrole-3-carboxylate

Potassium carbonate (1131.9 mg, 8.19 mmol) was added to a solution of compound 555a (500 mg, 2.73 mmol) and compound 555b (341 mg, 2.73 mmol) in acetonitrile (50 mL). The reaction mixture was heated to 50°C and stirred overnight until completion. Workup: The reaction mixture was added into water and extracted with ethyl acetate (2×50 mL). The organic phases were combined, washed with saturated brine, dried over anhydrous sodium sulfate, filtered, and concentrated under reduced pressure. The crude product was purified by column chromatography [TLC: PE/EA = 3:1; column elution: PE/EA = 100:0 - 85:15] to obtain compound 555c as a white solid (510 mg, yield 68.72%). LCMS [M+1]+ = 272.

### Step 2: Compound 555d

### Methyl 1-(2,5-dichloropyrimidin-4-yl)-1H-pyrrole-3-carboxylate

Compound 555c (1.1 g, 4.04 mmol) was added to a mixture of acetic acid (30 mL) and concentrated hydrochloric acid (15 mL). The reaction mixture was heated to 50°C and stirred for 10 hours until completion. Workup: The reaction mixture was added into water, and the aqueous phase was extracted with ethyl acetate (2×100 mL). The organic phases were combined, washed with saturated brine, dried over anhydrous sodium sulfate, filtered, and concentrated under reduced pressure. The crude product was purified by column chromatography [TLC: dichloromethane/methanol = 10:1; column elution: dichloromethane/methanol = 100:0 - 20:80] to obtain compound 555d as a white solid (315 mg, yield 30%). LCMS [M+1]⁺ = 258, 260.

### Step 3: Compound 555f

### 1-(2,5-Dichloropyrimidin-4-yl)-N-(cyanomethyl)-1H-pyrrole-3-carboxamide

2-(7-Azabenzotriazol-1-yl)-N,N,N',N'-tetramethyluronium hexafluorophosphate (375.7 mg, 0.99 mmol), N,N-diisopropylethylamine (212.5 mg, 1.65 mmol) and compound 555e (61.2 mg, 0.66 mmol) were added to a solution of compound 555d (170 mg, 0.66 mmol) in N,N-dimethylformamide (17 mL). The reaction mixture was stirred at room temperature for 10 minutes until completion. Workup: The reaction mixture was added into water, and the aqueous phase was extracted with ethyl acetate (3×100 mL). The organic phases were combined, washed with saturated brine, dried over anhydrous sodium sulfate, filtered, and concentrated under reduced pressure. The crude product was purified by column chromatography [TLC: PE/EA = 10:1; column elution: PE/EA = 100:0 - 50:50] to obtain compound 555f as a white solid (117 mg, yield 54%). LCMS [M+1]⁺ = 296, 298.

### Step 4: Compound 555

### 1-(5-Chloro-2-((2-methoxy-4-(4-methylpiperazin-1-yl)phenyl)amino)pyrimidin-4-yl)-N-(cyanomethyl)-1H-pyrrole-3-carboxamide

Compound 555f (20 mg, 0.07 mmol), compound 555g (15 mg, 0.07 mmol), palladium acetate (3 mg, 0.01 mmol), 4,5-bis(diphenylphosphino)-9,9-dimethylxanthene (15.6 mg, 0.03 mmol), cesium carbonate (44 mg, 0.14 mmol) and 1,4-dioxane (4 mL) were added to a 100 mL three-necked flask. The reaction mixture was purged with argon several times, heated to 100°C and stirred for 2 hours until completion. Workup: The reaction mixture was concentrated under reduced pressure. Ethyl acetate (100 mL) and water (100 mL) were added to the residue for liquid-liquid extraction. The organic phases were combined, washed with saturated brine, dried over anhydrous sodium sulfate, filtered, and concentrated under reduced pressure. The crude product was purified by preparative chromatography to obtain compound 555 as a yellow solid (3.2 mg, yield 9.8%). LCMS [M+1]⁺ = 481, 483.

¹H NMR (400 MHz, DMSO) δ 8.86 (t, J = 5.6 Hz, 1H), 8.71 (s, 1H), 8.53 (s, 1H), 8.16 (s, 1H), 8.14 (s, 1H), 7.53 (s, 1H), 7.41 (d, J = 8.7 Hz, 1H), 6.73 (dd, J = 3.2, 1.6 Hz, 1H), 6.63 (d, J = 2.4 Hz, 1H), 6.50 (dd, J = 8.7, 2.5 Hz, 1H), 4.27 (d, J = 5.5 Hz, 2H), 3.78 (s, 3H), 3.17-3.12 (m, 4H), 2.47 (d, J = 4.9 Hz, 4H), 2.24 (s, 3H).

The following compounds were synthesized in a similar manner to Example 9: N-(cyanomethyl)-1-(2-((2-methoxy-4-(1-methylpiperidin-4-yl)phenyl)amino)pyrimidin-4-yl)-1H-pyrrole-3-carboxamide (TDM-181156), a white solid (7.4 mg, yield 16.8%); 1-(5-chloro-2-((2-methoxy-4-(piperidin-4-yl)phenyl)amino)pyrimidin-4-yl)-N-(cyanomethyl)-1H-pyrrole-3-carboxamide (TDM-181157), a white solid (27.7 mg, yield 47%); and N-cyanomethyl-1-(2-methoxy-4-methylpyrrolidin-3-phenyl)amino)pyrimidin-4-ylpyrrole-3-carboxamide (TDM-181158), a white solid (6.2 mg, yield 3.4%).

| **TDM No.** | **Structure** | **LCMS** [M+1]⁺ | **¹H-NMR** |
|---|---|---|---|
| TDM-181156 | | 446.2 | ¹H NMR (400 MHz, DMSO) δ 8.86 (t, J = 5.6 Hz, 1H), 8.48 (d, J = 5.5 Hz, 1H), 8.31 - 8.26 (m, 2H), 8.22 (s, 1H), 7.91 (d, J = 8.2 Hz, 1H), 7.76 - 7.71 (m, 1H), 7.18 (d, J = 5.6 Hz, 1H), 6.95 (d, J = 1.6 Hz, 1H), 6.86 (dd, J = 8.2, 1.6 Hz, 1H), 6.76 (dd, J = 3.2, 1.6 Hz, 1H), 4.28 (d, J = 5.5 Hz, 2H), 3.85 (s, 3H), 2.93 (d, J = 11.3 Hz, 2H), 2.48 (s, 1H), 2.25 (s, 3H), 2.10 - 2.01 (m, 2H), 1.74 (dt, J = 12.1, 7.3 Hz, 4H). |
| TDM-181157 | | 446.2 | ¹H NMR (400 MHz, DMSO) δ 8.88 (t, J = 5.6 Hz, 1H), 8.81 (s, 1H), 8.60 (d, J = 3.1 Hz, 1H), 8.26 (s, 1H), 8.17 (t, J = 1.8 Hz, 1H), 7.70 (d, J = 8.2 Hz, 1H), 7.63 - 7.52 (m, 1H), 6.92 (d, J = 1.4 Hz, 1H), 6.83 (dd, J = 8.2, 1.5 Hz, 1H), 6.75 (dd, J = 3.2, 1.7 Hz, 1H), 4.27 (d, J = 5.5 Hz, 2H), 3.83 (s, 3H), 2.93 (t, J = 11.5 Hz, 4H), 2.79 (d, J = 12.2 Hz, 1H), 1.93 (d, J = 12.5 Hz, 2H), 1.79 (t, J = 11.2 Hz, 2H). |
| TDM-181158 | | 432.2 | ¹H NMR (400 MHz, DMSO) δ 8.87 (t, J = 5.4 Hz, 1H), 8.49 (d, J = 5.5 Hz, 1H), 8.30 (s, 2H), 8.24 (s, 1H), 7.92 (d, J = 8.2 Hz, 1H), 7.74 (s, 1H), 7.19 (d, J = 5.5 Hz, 1H), 6.99 (s, 1H), 6.92 (d, J = 8.2 Hz, 1H), 6.77 (s, 1H), 4.28 (d, J = 5.5 Hz, 2H), 3.38 (dd, J = 16.4, 8.2 Hz, 2H), 3.02 (t, J = 8.7 Hz, 1H), 2.79 (t, J = 7.0 Hz, 2H), 2.65-2.59 (m, 1H), 2.42 (s, 3H), 2.34-2.24 (m, 1H), 1.86 (dd, J = 12.7, 7.6 Hz, 1H). |

### Example 10 General Method for Synthesizing Compound 559 (TDM-181159)

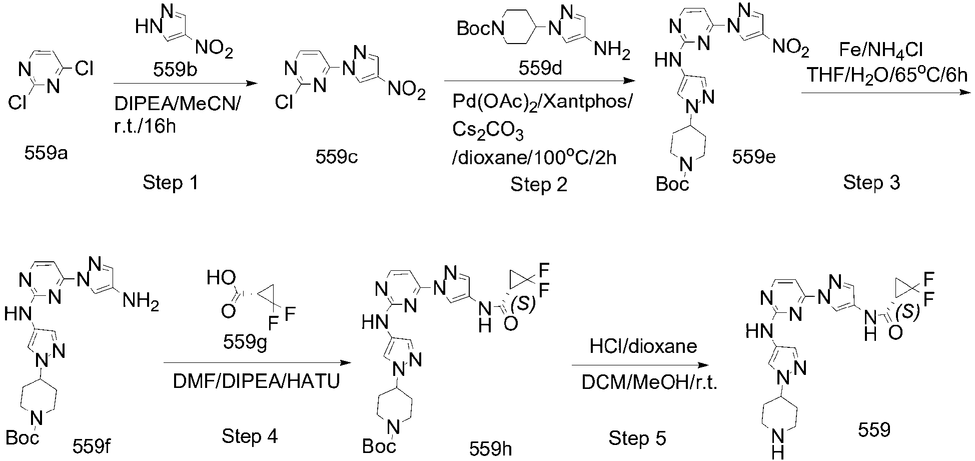

### Step 1: Compound 559c

### 2-Chloro-4-nitropyrazolopyrimidine

N,N-Diisopropylethylamine (2.84 g, 0.03 mol) was added to a mixture of compound 559a (3 g, 0.02 mol) and compound 559b (430.74 mg, 2.01 mmol) in acetonitrile (36 mL). The reaction mixture was stirred at room temperature for 16 hours. After completion of the reaction, the mixture was concentrated under reduced pressure. Water (100 mL) was added to the residue, which was extracted with ethyl acetate (3×50 mL). The organic layers were combined, washed with brine (50 mL), and dried over sodium sulfate. The filtrate was concentrated under reduced pressure, and the residue was purified by silica gel chromatography (petroleum ether/ethyl acetate = 0-76/24) to obtain compound 559c as a colorless solid (1.334 g, yield 29.6%). LCMS [M+1]⁺ = 226.1.

### Step 2: Compound 559e

### Tert-butyl 4-(4-(4-nitropyrazol-1-ylaminopyrimidin-1-ylamino)-1H-pyrazol-1-ylpiperidine-1-carboxylate

4,5-Bis(diphenylphosphino)-9,9-dimethylxanthene (53.3 mg, 0.09 mmol), palladium acetate (10.3 mg, 0.046 mmol) and cesium carbonate (149.9 mg, 0.46 mmol) were added to a mixture of compound 559c (50 mg, 0.23 mmol) and compound 559d (50.2 mg, 0.23 mmol) in 1,4-dioxane (2 mL). The mixture was degassed under vacuum, purged with argon, and stirred at 100°C for 2 hours. After completion of the reaction, the mixture was concentrated under reduced pressure. Water (30 mL) was added to the residue, which was basified with potassium carbonate solution and extracted with ethyl acetate (3×20 mL). The organic layers were combined and concentrated under reduced pressure. The residue was purified by silica gel column chromatography (petroleum ether/ethyl acetate = 0~50/50) to obtain compound 559e as a yellow solid (55.7 mg, yield 55.2%). LCMS [M+1-56]+ = 400.1.

### Step 3: Compound 559f

### Tert-butyl 4-(4-(4-(4-aminopyrazol-1-yl)aminopyrimidin-2-yl)amino-1H-pyrazol-1-yl)piperidine-1-carboxylate

Ammonium chloride (229.24 mg, 4.29 mmol), iron powder (239.35 mg, 4.29 mmol) and water (25 mL) were added to a solution of compound 559e (390.4 mg, 0.86 mmol) in tetrahydrofuran (25 mL). The mixture was stirred at 65°C for 6 hours. After completion of the reaction, the mixture was filtered and concentrated under reduced pressure. Water (60 mL) was added to the residue, which was extracted with ethyl acetate (3×40 mL). The organic layers were combined, washed with brine (50 mL), dried over sodium sulfate, and concentrated under reduced pressure. The residue was purified by silica gel column chromatography (dichloromethane / dichloromethane containing 10% methanol = 0-43/57) to obtain compound 559f as a white solid (203.7 mg, yield 55.7%). LCMS [M+1]⁺ = 426.2.

### Step 4: Compound 559h

### 4-(4-(4-(4-(2,2-difluorocyclopropane-1-carboxamido)-1H-pyrazol-1-ylaminopyrimidine-1H-pyrazol-1-ylaminopiperidine

2-(7-Azabenzotriazol-1-yl)-N,N,N',N'-tetramethyluronium hexafluorophosphate (107.2 mg, 0.28 mmol) and N,N-diisopropylethylamine (36.4 mg, 0.28 mmol) were added to a solution of compound 559g (34.4 mg, 0.28 mmol) in N,N-dimethylformamide (5 mL). The mixture was stirred at room temperature for 10 minutes. Compound 559f (80 mg, 0.19 mmol) was then added, and the mixture was stirred at room temperature for 1 hour. After completion of the reaction, the mixture was filtered and concentrated under reduced pressure. Water (30 mL) was added to the residue, which was extracted with ethyl acetate (5×20 mL). The organic layers were combined, washed with brine (3×30 mL), dried over sodium sulfate, and concentrated under reduced pressure. The residue was purified by silica gel column chromatography (dichloromethane / dichloromethane containing 10% methanol = 0-37/63) to obtain compound 559h as a yellow solid (84.3 mg, yield 86.6%). LCMS [M+1]+ = 530.3.

### Step 5: Compound 559

### 2,2-Difluoro-N-(1-2-((1-(piperidin-4-yl)-1H-pyrazol-4-ylamino)pyrimidin-4-yl)-1H-pyrazol-4-yl)cyclopropane-1-carboxamide

Hydrogen chloride in 1,4-dioxane (0.38 mL) was added to a solution of compound 559f (36.76 mg, 0.14 mmol) in solvent A (dichloromethane/methanol = 10:1, 3 mL). The reaction mixture was stirred at room temperature for 2 hours. After completion of the reaction, the mixture was concentrated under reduced pressure, and the residue was purified by preparative HPLC (formic acid) to obtain compound 559 (TDM-181159) as a white solid (18.1 mg, yield 49.5%). LCMS [M+1]⁺ = 430.1.

¹H NMR (400 MHz, MeOD) δ 8.94 (s, 1H), 8.54 (s, 1H), 8.40 (d, J = 5.5 Hz, 1H), 8.20 (s, 1H), 7.76 (s, 1H), 7.57 (s, 1H), 7.22 (d, J = 5.5 Hz, 1H), 4.51 (t, J = 11.1 Hz, 1H), 3.51 (dd, J = 7.8, 3.6 Hz, 2H), 3.21-3.12 (m, 2H), 2.73-2.64 (m, 1H), 2.38 (s, 2H), 2.24 (dd, J = 24.6, 12.9 Hz, 2H), 2.09 (dt, J = 13.7, 6.9 Hz, 1H), 1.96-1.86 (m, 1H).

### Example 11 General Method for Synthesizing Compound 569 (TDM-181169)

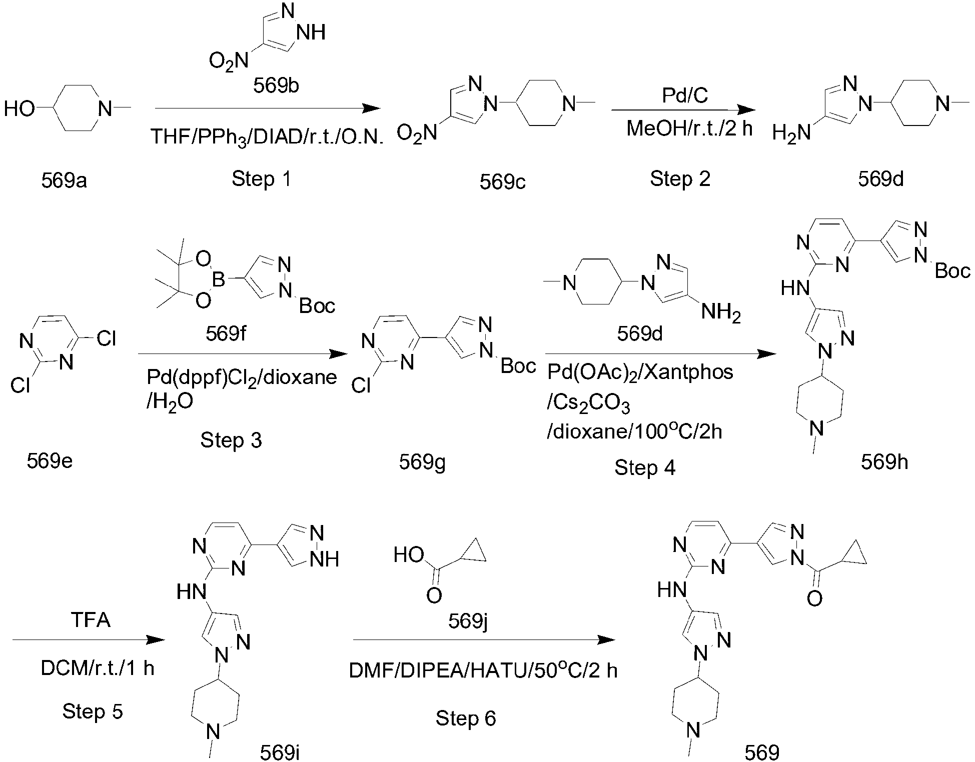

### Step 1: Compound 569c

### 1-Methyl-4-(4-nitro-1H-pyrazol-1-yl)piperidine

Under nitrogen protection, compound 569b (146.6 mg, 1.3 mmol), compound 569a (149.3 mg, 1.3 mmol), triphenylphosphine (682 mg, 2.6 mmol) and anhydrous tetrahydrofuran (10 mL) were added to a 100 mL three-necked flask. The mixture was cooled to 0°C, and diisopropyl azodicarboxylate (0.46 mL, 2.6 mmol) was added. The reaction mixture was stirred at room temperature overnight until completion. Workup: The reaction mixture was added into water and extracted with ethyl acetate (3×50 mL). The organic phases were combined, washed with saturated brine, dried over anhydrous sodium sulfate, filtered, and concentrated under reduced pressure. The crude product was purified by column chromatography [eluent: (dichloromethane/methanol = 10:1)/DCM = 0-40%] to obtain compound 569c as a yellow oil (110 mg, yield 40.3%). LCMS [M+1]⁺ = 211.

### Step 2: Compound 569d

### 1-(1-Methylpiperidin-4-yl)-1H-pyrazol-4-amine

Palladium on carbon (10 mg) was added to a solution of compound 569c (120 mg, 0.57 mmol) in methanol (10 mL). The mixture was purged with hydrogen several times and stirred at room temperature for 2 hours until completion. Workup: The reaction mixture was filtered, the cake was washed with methanol, and the filtrate was concentrated under reduced pressure to obtain compound 569d as a white solid (83.7 mg, yield 81.5%). LCMS [M+H]+ = 181.

### Step 3: Compound 569g

### Tert-butyl 4-(2-chloropyrimidin-4-yl)-1H-pyrazole-1-carboxylate

Compound 569e (500 mg, 3.36 mmol), compound 569f (988 mg, 3.36 mmol), [1,1'-bis(diphenylphosphino)ferrocene]dichloropalladium(II) (254.80 mg, 0.34 mmol), cesium carbonate (2189.3 mg, 6.72 mmol), 1,4-dioxane (25 mL) and water (2.5 mL) were added to a 100 mL three-necked flask. The mixture was purged with argon several times, heated to 70°C and stirred for 1 hour until completion. Workup: The reaction mixture was added into water and extracted with ethyl acetate (3×400 mL). The organic phases were combined, washed with saturated brine, dried over anhydrous sodium sulfate, and concentrated under reduced pressure. The crude product was purified by column chromatography [eluent: PE/EA = 0-30%] to obtain compound 569g as a white solid (588.1 mg, yield 62.4%). LCMS [M+H]⁺ = 281.

### Step 4: Compound 569h

### Tert-butyl 4-(2-((1-(1-methylpiperidin-4-yl)-1H-pyrazol-4-yl)amino)pyrimidin-4-yl)-1H-pyrazole-1-carboxylate

Compound 569g (504.8 mg, 1.8 mmol), compound 569d (389 mg, 2.16 mmol), palladium acetate (80.8 mg, 0.36 mmol), 4,5-bis(diphenylphosphino)-9,9-dimethylxanthene (416.6 mg, 0.72 mmol), cesium carbonate (1173 mg, 3.6 mmol) and 1,4-dioxane (25 mL) were added to a 100 mL three-necked flask. The mixture was purged with argon several times, heated to 100°C and stirred for 2 hours until completion. Workup: The reaction mixture was concentrated under reduced pressure. Ethyl acetate (100 mL) and water (100 mL) were added for liquid-liquid extraction. The organic phases were combined, washed with saturated brine, dried over anhydrous sodium sulfate, and concentrated under reduced pressure. The crude product was purified by column chromatography [eluent: (dichloromethane/methanol = 10:1)/DCM = 0-100%] to obtain compound 569h as a yellow solid (225.8 mg, yield 29%). LCMS [M+H]⁺ = 425.

### Step 5: Compound 569i

### N-(1-(1-Methylpiperidin-4-yl)-1H-pyrazol-4-yl)-4-(1H-pyrazol-4-yl)pyrimidin-2-amine

Trifluoroacetic acid (2.2 mL) was added to a solution of compound 569h (225.8 mg, 0.53 mmol) in dichloromethane (20 mL). The reaction mixture was stirred at room temperature for 1 hour until completion. Workup: The reaction mixture was added into water and extracted with ethyl acetate (2×100 mL). The aqueous layers were combined, basified to alkaline pH, and extracted with ethyl acetate (2×100 mL). The organic phases were combined, washed with saturated brine, dried over anhydrous sodium sulfate, and concentrated under reduced pressure to obtain compound 569i as a white solid (172.5 mg, yield 92%). LCMS [M+H]⁺ = 325.

### Step 6: Compound 569

### Cyclopropyl(4-(2-((1-(1-methylpiperidin-4-yl)-1H-pyrazol-4-yl)amino)pyrimidin-4-yl)-1H-pyrazol-1-yl)methanone

2-(7-Azabenzotriazol-1-yl)-N,N,N',N'-tetramethyluronium hexafluorophosphate (270 mg, 0.71 mmol), N,N-diisopropylethylamine (92 mg, 0.71 mmol) and compound 569i (152.5 mg, 0.47 mmol) were added to a solution of compound 569j (61.1 mg, 0.71 mmol) in N,N-dimethylformamide (5 mL). The reaction mixture was heated to 50°C and stirred for 2 hours until completion. Workup: The reaction mixture was added into water and extracted with ethyl acetate (3×100 mL). The organic phases were combined, washed with saturated brine, dried over anhydrous sodium sulfate, and concentrated under reduced pressure. The crude product was purified by column chromatography [eluent: (dichloromethane/methanol = 10:1)/DCM = 0-50%], then recrystallized from methanol to obtain compound 569 as a white solid (11.7 mg, yield 9.2%). LCMS [M+H]⁺ = 393.2.

¹H NMR (400 MHz, DMSO-d6) δ 9.56 (s, 1H), 9.34 (s, 1H), 9.12 (s, 1H), 8.54 (s, 1H), 8.46 (d, J = 5.1 Hz, 1H), 8.04 (s, 1H), 7.62 (s, 1H), 7.28 (d, J = 5.1 Hz, 1H), 4.48 (s, 1H), 3.57 (d, J = 12.1 Hz, 2H), 3.12 (ddd, J = 12.3, 7.8, 4.6 Hz, 3H), 2.85 (s, 3H), 2.21 (d, J = 31.1 Hz, 4H), 1.35-1.12 (m, 4H).

### Example 12 General Method for Synthesizing Compound 570 (TDM-181170)

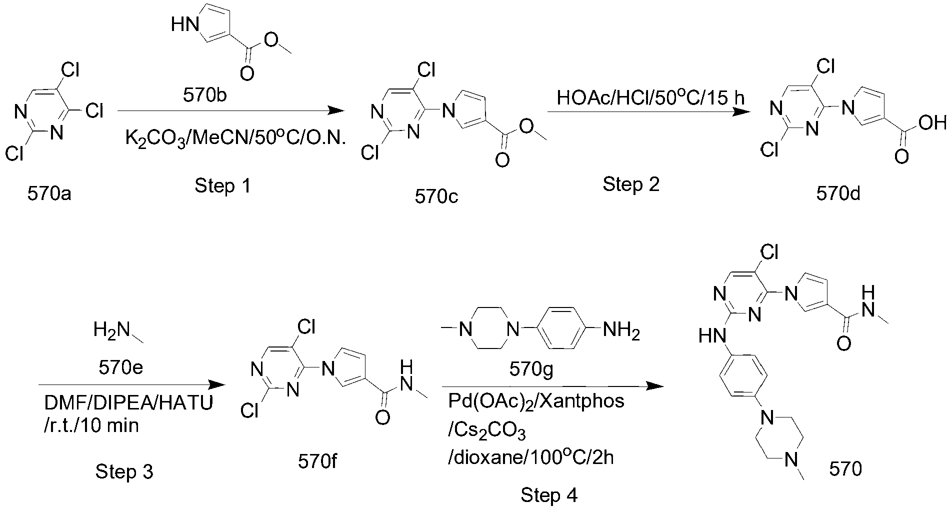

### Step 1: Compound 570c

### Methyl 1-(2,5-dichloropyrimidin-4-yl)-1H-pyrrole-3-carboxylate

Potassium carbonate (2.21 g, 16 mmol) was added to a mixture of compound 570a (1.46 g, 8 mmol) and compound 570b (1 g, 8 mmol) in acetonitrile (100 mL). The reaction mixture was heated to 50°C and stirred overnight until completion. Workup: The reaction mixture was added into water (150 mL) and extracted with ethyl acetate (2×200 mL). The organic phases were combined, washed with saturated brine, dried over anhydrous sodium sulfate, and concentrated under reduced pressure. The crude product was purified by column chromatography [TLC: PE/EA = 3:1; column elution: PE/EA = 100:0-85:15] to obtain compound 570c as a white solid (1.89 g, yield 87.5%). LCMS [M+1]⁺ = 272.

### Step 2: Compound 570d

### 1-(2,5-Dichloropyrimidin-4-yl)-1H-pyrrole-3-carboxylic acid

Concentrated hydrochloric acid (20 mL) was added to a solution of compound 570c (1.89 g, 6.95 mmol) in acetic acid (40 mL). The reaction mixture was heated to 50°C and stirred for 15 hours until completion. Workup: The reaction mixture was added into water and extracted with ethyl acetate (2×100 mL). The organic phases were combined, washed with saturated brine, dried over anhydrous sodium sulfate, and concentrated under reduced pressure. The crude product was purified by column chromatography [eluent: (dichloromethane/methanol = 10:1)/DCM = 0-15%] to obtain compound 570d as a white solid (495 mg, yield 26.7%). LCMS [M+1]⁺ = 258, 260.

### Step 3: Compound 570f

### 1-(2,5-Dichloropyrimidin-4-yl)-N-methyl-1H-pyrrole-3-carboxamide

2-(7-Azabenzotriazol-1-yl)-N,N,N',N'-tetramethyluronium hexafluorophosphate (353.6 mg, 0.93 mmol), N,N-diisopropylethylamine (200 mg, 1.55 mmol) and compound 570e (41.8 mg, 0.62 mmol) were added to a solution of compound 570d (158.6 mg, 0.62 mmol) in N,N-dimethylformamide (15 mL). The reaction mixture was stirred at room temperature for 10 minutes until completion. Workup: The reaction mixture was added into water and extracted with ethyl acetate (3×100 mL). The organic phases were combined, washed with saturated brine, dried over anhydrous sodium sulfate, and concentrated under reduced pressure. The crude product was purified by column chromatography [eluent: (dichloromethane/methanol = 10:1)/DCM = 0-15%] to obtain compound 570f as a yellow solid (129.8 mg, yield 65%). LCMS [M+1]⁺ = 271, 273.

### Step 4: Compound 570

### 1-(5-Chloro-2-((4-(4-methylpiperazin-1-yl)phenyl)amino)pyrimidin-4-yl)-N-methyl-1H-pyrrole-3-carboxamide

Compound 570f (104.8 mg, 0.39 mmol), compound 570g (88.7 mg, 0.46 mmol), palladium acetate (17.4 mg, 0.08 mmol), 4,5-bis(diphenylphosphino)-9,9-dimethylxanthene (89.6 mg, 0.16 mmol), cesium carbonate (252.2 mg, 0.77 mmol) and 1,4-dioxane (20 mL) were added to a 100 mL three-necked flask. The mixture was purged with argon several times, heated to 100°C and stirred for 2 hours until completion. Workup: The reaction mixture was concentrated under reduced pressure. The crude product was purified by column chromatography [eluent: (dichloromethane/methanol = 10:1)/DCM = 0-50%], then further purified by preparative chromatography to obtain compound 570 as a yellow solid (48.9 mg, yield 24%). LCMS [M+1]⁺ = 426.2.

¹H NMR (400 MHz, DMSO-d6) δ 9.79 (s, 1H), 8.59 (s, 1H), 8.16 (s, 1H), 8.09 (q, J = 2.4, 2.0 Hz, 2H), 7.56 (dd, J = 3.3, 2.3 Hz, 1H), 7.54-7.46 (m, 2H), 6.98-6.86 (m, 2H), 6.73 (dd, J = 3.3, 1.7 Hz, 1H), 3.08 (dd, J = 6.4, 3.6 Hz, 4H), 2.74 (d, J = 4.5 Hz, 3H), 2.46 (d, J = 4.9 Hz, 4H), 2.23 (s, 3H).

1-(5-Chloro-2-((4-(4-methylpiperazin-1-yl)phenyl)amino)pyrimidin-4-yl)-N-(cyclopropylmethyl)-1H-pyrrole-3-carboxamide (TDM-181171) was synthesized in a similar manner to Example 12 as a yellow solid (65 mg, yield 26%).

| **TDM No.** | **Structure** | **LCMS** [M+1]⁺ | **¹H-NMR** |
|---|---|---|---|
| TDM-181171 | | 466.2 | ¹H NMR (400 MHz, DMSO-d6) δ 9.80 (s, 1H), 8.60 (s, 1H), 8.22 (t, J = 5.8 Hz, 1H), 8.15 (s, 1H), 8.13 (t, J = 2.0 Hz, 1H), 7.57 (dd, J = 3.3, 2.2 Hz, 1H), 7.54 - 7.47 (m, 2H), 6.96 - 6.87 (m, 2H), 6.77 (dd, J = 3.3, 1.7 Hz, 1H), 3.10 (q, J = 6.0, 5.1 Hz, 6H), 2.49 - 2.45 (m, 4H), 2.24 (s, 3H), 1.07 - 0.95 (m, 1H), 0.49 - 0.38 (m, 2H), 0.26 - 0.19 (m, 2H). |

### Example 13 General Method for Synthesizing Compound 577 (TDM-181177)

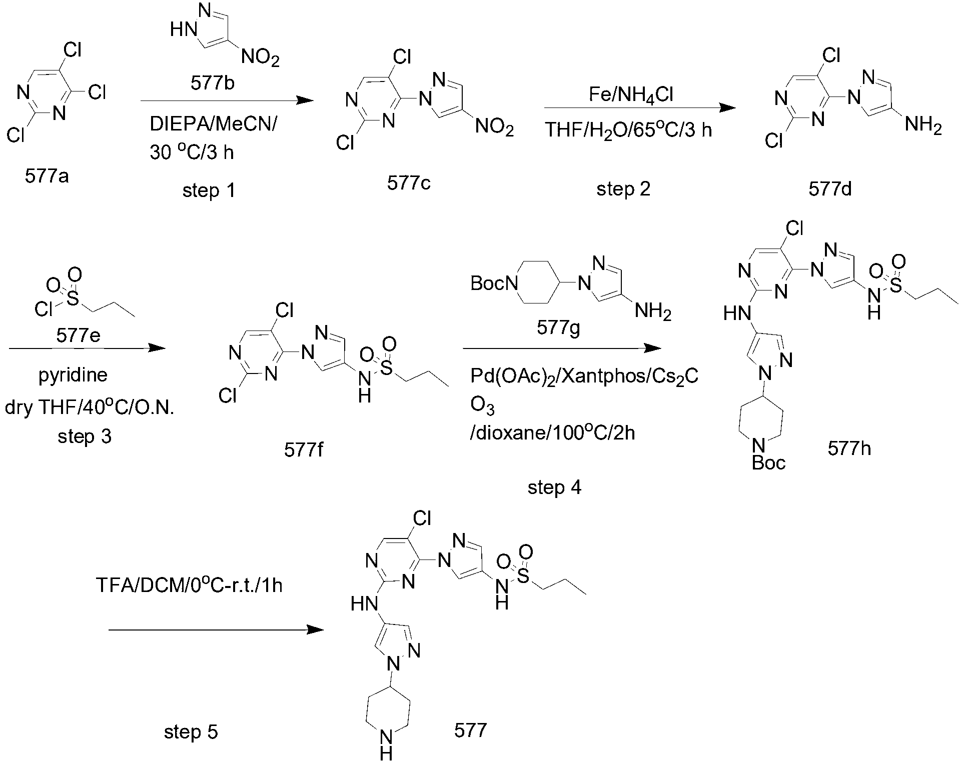

### Step 1: Compound 577c

### 2,5-Dichloro-4-(4-nitropyrazol-1-yl)pyrimidine

Compound 577b (0.6 g, 0.0052 mol) was added to a solution of compound 577a (1.02 g, 0.006 mol) in acetonitrile (20 mL), followed by the addition of N,N-diisopropylethylamine (0.78 g, 0.006 mol). The reaction mixture was heated to 30°C and stirred for 3 hours. LCMS [M+1]+ = 260, indicating the reaction was complete. Workup: The reaction mixture was concentrated to dryness. Ethyl acetate (120 mL) and water (60 mL) were added to the crude product for layer separation. The separated organic phase was washed with water and saturated brine, dried over anhydrous sodium sulfate. The crude product was purified by column chromatography [eluent: EA/PE = 0-30%] to obtain compound 577c as a white solid (1.02 g, yield 65.4%). LCMS [M+1]⁺ = 260.

### Step 2: Compound 577d

### 1-(2,5-Dichloropyrimidin-4-yl)-1H-pyrazol-4-amine

Ammonium chloride (944.10 mg, 17.65 mmol), iron powder (985.64 mg, 17.65 mmol) and water (30 mL) were added to a solution of compound 577c (917.80 mg, 3.53 mmol) in tetrahydrofuran (30 mL). The reaction mixture was heated to 65°C and stirred for 3 hours. LCMS [M+1]⁺ = 230.1, indicating the reaction was complete. Workup: The mixture was filtered. Ethyl acetate (100 mL) and water (50 mL) were added to the organic phase for layer separation. The separated organic phase was washed with water and saturated brine, dried over anhydrous sodium sulfate. The crude product was purified by column chromatography [eluent: EA/PE = 0-50%] to obtain compound 577d as a yellow solid (741 mg, yield 82%). LCMS [M+1]⁺ = 230.1.

### Step 3: Compound 577f

### N-(1-(2,5-Dichloropyrimidin-4-yl)-1H-pyrazol-4-yl)propane-1-sulfonamide

Pyridine (275.07 mg, 3.48 mmol) and compound 577e (488.80 mg, 3.43 mmol) were added to a solution of compound 577d (400 mg, 1.74 mmol) in anhydrous tetrahydrofuran (20 mL) at 0°C. The reaction mixture was stirred at room temperature for 1 hour, purged with N₂ several times, then heated to 40°C and stirred overnight. LCMS [M+1]⁺ = 336, indicating the reaction was complete. Workup: The reaction mixture was concentrated to dryness. Ethyl acetate (100 mL) and water (50 mL) were added to the crude product for layer separation. The separated organic phase was washed with water and saturated brine, dried over anhydrous sodium sulfate. The crude product was purified by column chromatography [eluent: EA/PE = 0-40%] to obtain compound 577f as a yellow liquid (531 mg, yield 86%). LCMS [M+1]+ = 336.

### Step 4: Compound 577h

### Tert-butyl 4-((5-chloro-4-(4-(propylsulfonamido)-1H-pyrazol-1-yl)pyrimidin-2-ylamino)-1H-pyrazol-1-yl)piperidine-1-carboxylate

Compound 577g (79.10 mg, 0.297 mmol), palladium acetate (13.34 mg, 0.059 mmol), 4,5-bis(diphenylphosphino)-9,9-dimethylxanthene (68.74 mg, 0.119 mmol) and cesium carbonate (193.53 mg, 0.594 mmol) were added to compound 577f (100 mg, 0.297 mmol). The reaction mixture was purged with argon several times, then anhydrous 1,4-dioxane (10 mL) was added. The mixture was purged with argon three times, heated to 100°C and stirred for 2 hours. LCMS [M-100]⁺ = 466.2, indicating the reaction was complete. Workup: Water (30 mL) and ethyl acetate (60 mL) were added to the reaction mixture for layer separation. The separated organic phase was washed with water and saturated brine, dried over anhydrous sodium sulfate. The crude product was purified by column chromatography [eluent: EA/PE = 0-50%] to obtain compound 577h as a yellow liquid (41 mg, yield 24.39%). LCMS [M-100]⁺ = 466.2.

### Step 5: Compound 577

### N-(1-(5-Chloro-2-(1-(piperidin-4-yl)-1H-pyrazol-4-ylamino)pyrimidin-4-yl)-1H-pyrazol-4-yl)propane-1-sulfonamide

Trifluoroacetic acid (0.3 mL) was added to a solution of compound 577h (31.9 mg, 0.06 mmol) in dichloromethane (3 mL) at 0°C. The reaction mixture was warmed to room temperature and stirred for 1 hour. LCMS [M+1]⁺ = 466.1, indicating the reaction was complete. Workup: The reaction mixture was concentrated to dryness to give a crude product, which was further purified by preparative chromatography to obtain compound 577 as a yellow solid (19.8 mg, yield 70.82%). LCMS [M+1]⁺ = 466.1.

¹H NMR (400 MHz, DMSO) δ 9.99 (s, 1H), 8.59 (s, 1H), 8.29 (d, J = 50.1 Hz, 2H), 7.93 (s, 1H), 7.77 (s, 1H), 7.58 (s, 1H), 4.39 (s, 1H), 3.29 (s, 2H), 3.13-3.07 (m, 2H), 2.93 (t, J = 11.1 Hz, 2H), 2.17-1.95 (m, 5H), 1.78-1.65 (m, 2H), 0.97 (t, J = 7.4 Hz, 3H).

### Example 14 General Procedure for the Synthesis of Compound 579 (TDM-181179)

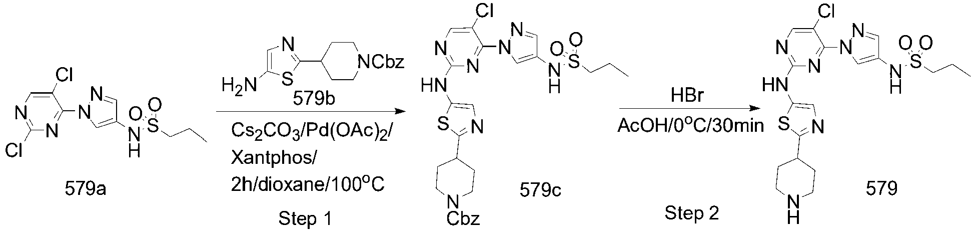

### Step 1: Compound 579c

### Benzyl 4-(5-((5-chloro-4-(4-(propylsulfonamido)-1H-pyrazol-1-yl)pyrimidin-2-yl)amino)thiazol-2-yl)piperidine-1-carboxylate

1,4-Dioxane (5 mL) was added to a mixture of compound 579a (39 mg, 0.117 mmol), compound 579b (37 mg, 0.115 mmol), palladium acetate (5 mg, 0.023 mmol), xantphos (27 mg, 0.047 mmol) and cesium carbonate (76 mg, 0.233 mmol). The mixture was degassed under vacuum, purged with argon several times, heated to 100°C and stirred for 2 hours. The mixture was concentrated under reduced pressure, and the residue was purified by silica gel chromatography (dichloromethane/10% methanol in dichloromethane = 0-25/75) to obtain compound 579c as a brown solid (40 mg, yield 55.4%). LCMS [M+1]⁺ = 617.

### Step 2: Compound 579

### N-(1-(5-Chloro-2-((2-(piperidin-4-yl)thiazol-5-yl)amino)pyrimidin-4-yl)-1H-pyrazol-4-yl)propane-1-sulfonamide

Compound 579c (40 mg, 0.065 mmol) was dissolved in HBr-AcOH (2 mL) at 0°C, and the mixture was stirred at 0°C for 30 minutes. The mixture was concentrated under reduced pressure at 45°C, and the residue was purified by preparative HPLC (formic acid) to obtain compound 579 (TDM-181179) as a yellow solid (7 mg, yield 22.3%). LCMS [M+1]⁺ = 483.

¹H NMR (400 MHz, DMSO) δ 8.68 (s, 1H), 8.48 (s, 1H), 8.37 (s, 1H), 7.79 (s, 1H), 7.41 (s, 1H), 3.20 (d, J = 12.2 Hz, 2H), 3.16 (s, 1H), 3.12-3.07 (m, 2H), 2.85 (t, J = 11.4 Hz, 2H), 2.52 (s, 1H), 2.09 (d, J = 12.2 Hz, 2H), 1.84 (s, 2H), 1.71 (dq, J = 14.9, 7.5 Hz, 2H), 0.96 (t, J = 7.4 Hz, 3H).

### Test Example: Enzyme Activity Inhibition Test of NUAK1/NUAK2 Kinase Inhibitors

The inhibitory activity of the pyrimidinamine small molecules involved in the present application against NUAK1 and NUAK2 kinases was determined using a kinase activity assay based on the P81 filter paper binding hot-spot technique (Nat Biotechnol. 2011, 29:1039). A brief description is as follows:

The assay buffer system contained 20 mM Hepes (pH 7.5), 10 mM MgCl₂, 1 mM EGTA, 0.01% Brij35 (L23 polyoxyethylene lauryl ether), 0.02 mg/mL BSA (bovine serum albumin), 0.1 mM Na₃VO₄, 2 mM DTT, and 1% DMSO.

### Test Steps:

1. A 20 µM substrate solution was prepared with freshly prepared test buffer and CHKtide peptide fragments (Sanchez Y. Science. 1997, 277: 1497-1501) were used as a substrate.
2. Kinases were added and mixed gently (a final concentration of NUAK1 was 10 nM; and a final concentration of NUAK2 was 50 nM).
3. Test compounds dissolved in 100% DMSO were each added to the above kinase reaction mixture solution via Acoustic Droplet Ejection (Echo550; nanoliter) and the mixture was incubated for 5 minutes at room temperature.
4. ³³P-labeled ATP (a ratio of unlabeled ATP to labeled ATP was 25:1 or 2.5:1) was added.
5. The mixture was incubated for 2 hours at room temperature.
6. Kinase activity was tested using the P81 filter paper combined with hotspot technology.

The IC₅₀ values of the test compounds against NUAK1/NUAK2 were calculated according to the above test. For specific results, see Table 1.

The IC₅₀ values were calculated by using a formula derived from sigmoidal dose-response curve (variable slope):
Y = Bottom + (Top-Bottom)/(1+10^((LogEC50-X)*Slope, where X is the logarithm of compound concentration, Y is the response (inhibition rate of kinase activity), and Y rises from bottom to top along the sigmoidal curve as the concentration increases.

**Table 1: IC₅₀ (nM) of Test Compounds of the Present Application against NUAK1/NUAK2**

| Test Compound | NUAK1 | NUAK2 |
|---|---|---|
| TDM-181134 | 122 | 622 |
| TDM-181135 | 34 | 265 |
| TDM-181139 | 474 | 991 |
| TDM-181140 | 305 | 210 |
| TDM-181146 | 260 | 602 |
| TDM-181147 | 19 | 77 |
| TDM-181152 | 977 | Untested |
| TDM-181153 | 84 | 107 |
| TDM-181154 | 58 | 239 |
| TDM-181155 | 43 | 69 |
| TDM-181156 | 607 | Untested |
| TDM-181157 | 194 | 487 |
| TDM-181158 | 394 | Untested |
| TDM-181159 | 337 | 419 |
| TDM-181169 | 209 | 495 |
| TDM-181170 | 14 | 78 |
| TDM-181171 | 9 | 50 |
| TDM-181177 | 9 | 72 |
| TDM-181179 | 6 | 42 |

As can be seen from the results in Table 1, the pyrimidinamine-based compounds of the present application exhibit excellent inhibitory activity against both NUAK1 and NUAK2, and are dual-target small-molecule kinase inhibitors. The IC₅₀ values of the compounds against NUAK1/NUAK2 can reach several nM/several tens of nM. Therefore, the above experiments demonstrate that the pyrimidinamine-based compounds of the present application can be used as NUAK1/NUAK2 inhibitors.

The preferred embodiments of the present disclosure have been described in detail above. However, the present disclosure is not limited to the specific details in the above embodiments. Within the scope of the technical concept of the present disclosure, various simple modifications can be made to the technical solutions of the present disclosure, and all such simple modifications fall within the scope of protection of the present disclosure.

In addition, it should be noted that the various specific technical features described in the above specific embodiments may be combined in any suitable manner without contradiction. To avoid unnecessary repetition, the present disclosure does not separately describe every possible combination.

Moreover, any combination of the various embodiments of the present disclosure may be made, provided that such combination does not depart from the spirit of the present disclosure, and such combinations shall also be regarded as the content disclosed in the present disclosure.

## Claims

1. A pyrimidinamine-based compound, wherein the pyrimidinamine-based compound is a compound represented by Formula I, or a stereoisomer, a tautomer, an isotopic derivative, a hydrate, a solvate, a prodrug, or a pharmaceutically acceptable salt thereof, 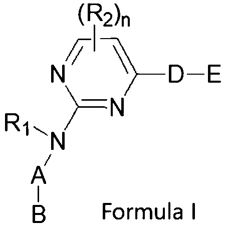
wherein A is a phenyl group optionally substituted by m R₃ groups, or a 5-membered heteroaryl group optionally substituted by p R₄ groups;
B is an optionally substituted 3- to 10-membered cycloalkyl group or an optionally substituted 3- to 10-membered heterocycloalkyl group;
the substituent on A or B is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;
D is 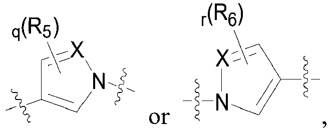 X is N or CH, and when X is CH, the H atom may be substituted by R₅ or R₆;
E is -C(O)-NHR₇R₈, -C(O)-R₇R₈, -NR₇-C(O)R₈, -NR₇-S(O)₂-R₈, an optionally substituted C3-C8 heterocycloalkyl-S(O)₂-R₈, or an optionally substituted C3-C8 cycloalkyl-S(O)₂-R₈; R₈ is an optionally substituted C1-C8 alkyl group, an optionally substituted C3-C8 cycloalkyl group, or an optionally substituted C3-C8 cycloalkyl C1-C8 alkyl group, and the substituent on the cycloalkyl group or the heterocycloalkyl group is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group.
R₁ and R₇ are each independently H or an optionally substituted C1-C8 alkyl group;
R₂, R₃, R₄, R₅, and R₆ are each independently halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, or an optionally substituted C1-C8 alkylamino group;
the substituent on the C1-C8 alkyl group is one or more selected from the group consisting of halogen, an amino group, a hydroxyl group, a nitro group, a cyano group, and a sulfhydryl group; and
n is 0, 1, or 2; m is 0, 1, 2, 3, or 4; and p, q, and r are each 0, 1, 2, or 3.

2. The compound according to claim 1, wherein E is one of the following structures:

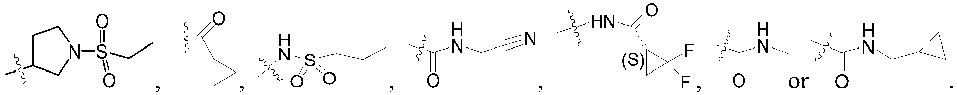

3. The compound according to any one of claims 1-2, wherein B is an optionally substituted 3-to 6-membered heterocycloalkyl group.

4. The compound according to claim 3, wherein B is an optionally substituted 5- to 6-membered heterocycloalkyl group.

5. The compound according to claim 4, wherein B is an optionally substituted piperidinyl group, an optionally substituted piperazinyl group, or an optionally substituted pyrrolidinyl group.

6. The compound according to claim 5, wherein B is one of the following formulae: 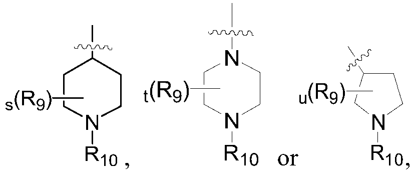
wherein R₉ is halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, or an optionally substituted C1-C8 alkylamino group;
R₁₀ is H or an optionally substituted C1-C8 alkyl group;
the substituent on the C1-C8 alkyl group is one or more selected from the group consisting of halogen, an amino group, a hydroxyl group, a nitro group, a cyano group, and a sulfhydryl group; and
s and t are each 0, 1, 2, 3, or 4; and u is 0, 1, 2, or 3.

7. The compound according to claim 6, wherein B is one of the following formulae:

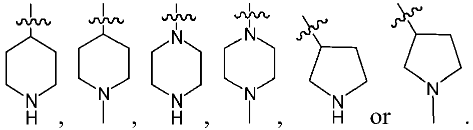

8. The compound according to any one of claims 1 to 7, wherein A is one of the following optionally substituted moieties:

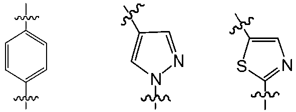

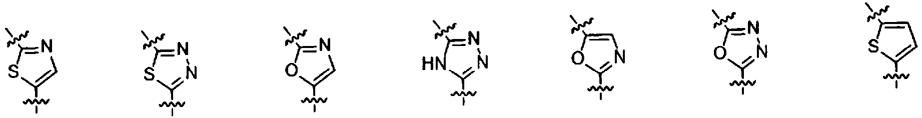

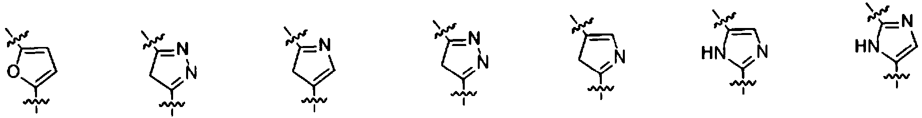

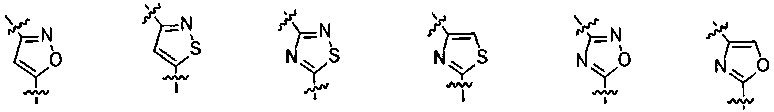

9. The compound according to claim 8, wherein A is an optionally substituted phenyl group.

10. The compound according to claim 9, wherein A is substituted with a methoxy group, preferably at the ortho-position to the amino group.

11. The compound according to any one of claims 1 to 10, wherein R₂ is chloro, preferably at the para-position to the amino group.

12. The compound according to any one of claims 1 to 11, wherein the compound represented by Formula I is one of the following compounds:

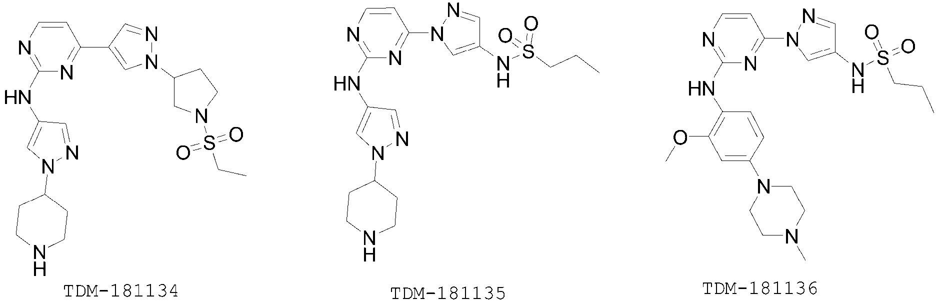

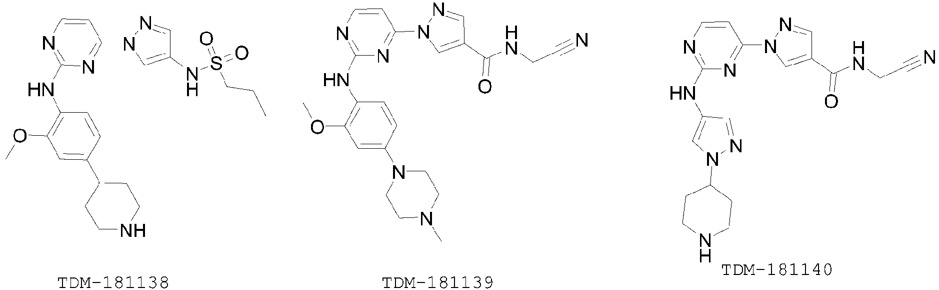

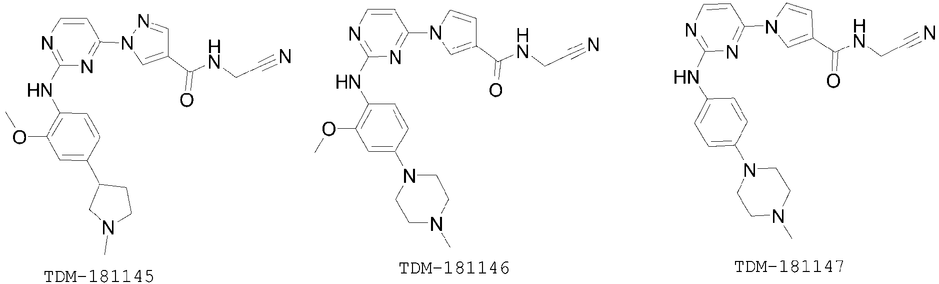

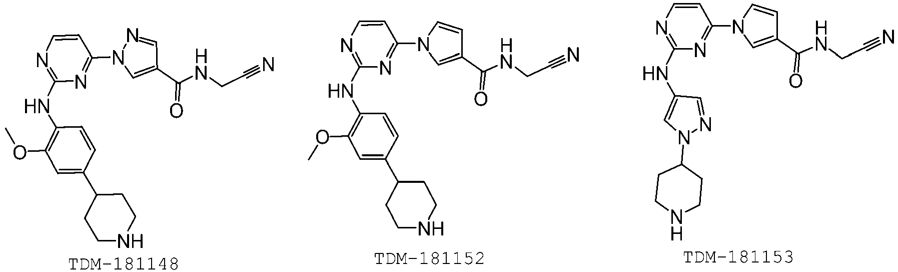

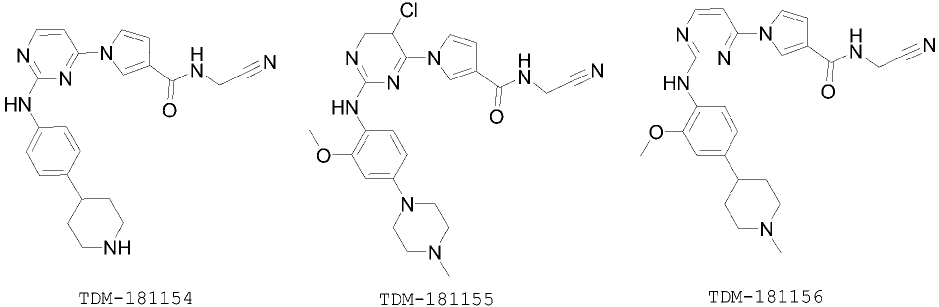

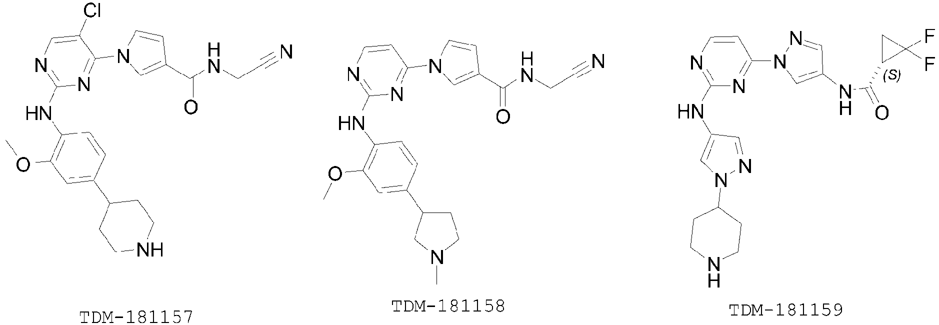

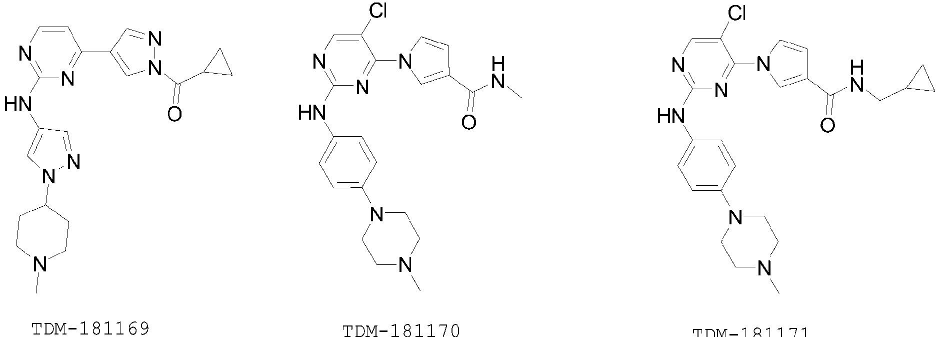

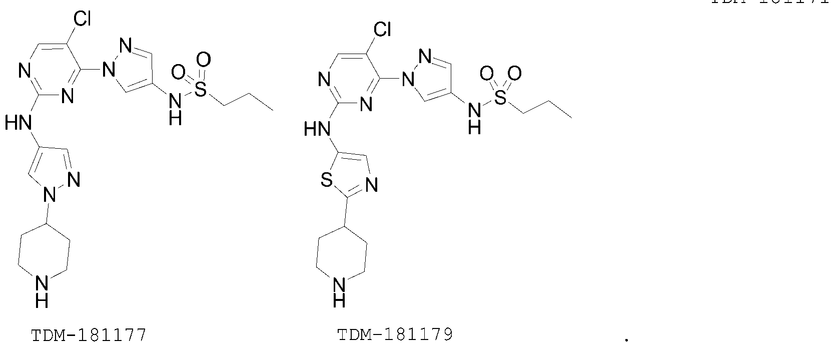

13. The compound according to any one of claims 1 to 12, wherein the pharmaceutically acceptable salt is a formate salt.

14. The compound according to any one of claims 1 to 13, wherein the isotopic derivative is a deuterated compound.

15. A method for preparing the compound according to any one of claims 1 to 14, comprising:
preparing the compound of Formula I from a compound of Formula II and a compound of Formula III; or
when D is 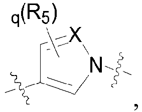 the compound of Formula I may alternatively be prepared in the following procedure: preparing a compound of Formula V from a compound of Formula IV and a compound of Formula II, and then preparing the compound of Formula I from the compound of Formula V;
optionally, the preparation process comprises necessary protection and deprotection steps; 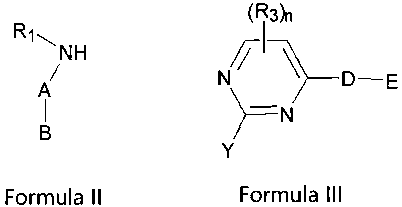 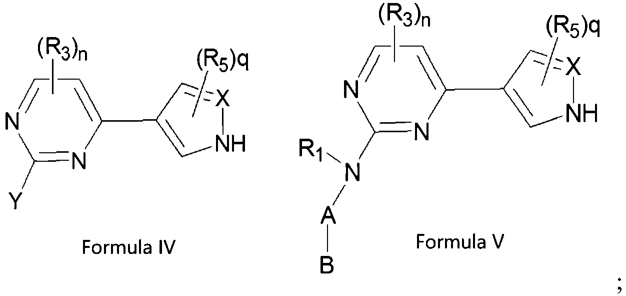
wherein Y is a leaving group, preferably Y is halogen, more preferably Y is Cl, and other groups are as defined in claims 1 to 14.

16. A pharmaceutical composition, wherein the pharmaceutical composition comprises the compound according to any one of claims 1 to 14 as an active ingredient.

17. The pharmaceutical composition according to claim 16, wherein the pharmaceutical composition comprises a pharmaceutically acceptable carrier or excipient.

18. Use of the compound according to any one of claims 1 to 14 in preparation of a NUAK1 or NUAK2 inhibitor.

19. Use of the compound according to any one of claims 1 to 14 in preparation of a medicament, wherein the compound is used for the prevention or treatment of the following diseases by inhibiting NUAK1 or NUAK2: neuropsychiatric diseases, metabolic diseases, tumors, visceral fibrotic diseases, or skin fibrotic diseases.

20. The use according to claim 19, wherein the disease is Parkinson's disease, Alzheimer's disease, diabetes, hyperlipidemia, obesity, liver cancer, leukemia, lymphoma, tumor metastasis, liver cirrhosis, renal fibrosis, pulmonary fibrosis, post-myocarditis sequelae, scleroderma, keloids, or hypertrophic scar; or the compound is used solely for alleviating a traumatic or postoperative scar.
